# EUROPEAN PATENT APPLICATION

(11) **EP 4 733 300 A1**
(43) Date of publication of application: **29.04.2026**
(21) Application number: 24826322.0
(22) Date of filing: 24.06.2024
(51) Int. Cl.: C07D 405/12, A61K 31/4184, A61P 35/00

(54) **NOVEL CHROMENE-BENZIMIDAZOLE DERIVATIVE OR PHARMACEUTICALLY ACCEPTABLE SALT THEREOF, AND USE THEREOF**

(30) Priority: 23.06.2023 KR 20230081298; 21.06.2024 KR 20240081292
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08226 (KR); KIM, Yoon Jae, Paju-si Gyeonggi-do 10887 (KR); JUNG, Eun-Sun, Incheon 21122 (KR); KANG, Yong Koo, Seoul 04426 (KR); PARK, Min Su, Seoul 08238 (KR); KIM, Seong Jae, Seoul 08307 (KR); LEE, Sang Yoon, Seoul 04765 (KR)
(74) Representative: ZBM Patents - Zea, Barlocci & Markvardsen
(86) International application number: PCT/KR2024/008662
(87) International publication number: WO 2024/263007

(57) **Abstract**

The present invention relates to a chromene-benzimidazole derivative or a pharmaceutically acceptable salt thereof, and a composition containing the derivative as an active ingredient for preventing or treating cancer. The chromene-benzimidazole derivative of the present invention is activated in cancer cells to inhibit tubulin polymerization and suppress the C-terminal domain of Hsp90, thereby blocking the cell cycle of cancer cells, inducing apoptosis, and inhibiting the activity of oncoproteins, and thus can be used as a novel dual-target anticancer drug for preventing or treating cancer, preferably for preventing or treating triple-negative breast cancer.

## Description

### TECHNICAL FIELD

The disclosure relates to a chromene-benzimidazole derivative or a pharmaceutically acceptable salt thereof, and uses thereof.

### BACKGROUND ART

Triple-negative breast cancer (TNBC; ER-, PR-, HER2-) patients account for 10 to 15% of all breast cancer patients, and lack hormone receptors (ER (estrogen receptor), PR (progesterone receptor)) and HER2 protein, and thus do not benefit from hormone therapy or HER2-targeted therapeutics. Currently, the standard treatment for triple-negative breast cancer relies entirely on general cytotoxic anticancer agents (Taxane-based or Anthracycline-based). Since there are no established targeted therapeutics, treatment strategies are not as diverse compared to other subtypes of breast cancer. More seriously, after surgery or chemotherapy, recurrence appears within 2 to 3 years in most patients, and metastasis to other organs such as the lung, liver, brain, and bone is easily induced, affecting the decrease in patient survival rate.

The 5-year overall survival rate of patients diagnosed with stage-III is 55% or less, and patients with advanced-stage cancer already progressed to metastasis have a very low 5-year overall survival rate of 30% or less. Most of these patients ultimately die within several years, making it a very serious disease.

Microtubules are a major component of the cytoskeleton and are composed of tubulin heterodimers consisting of an α subunit and a β subunit. Microtubules perform various cellular functions such as intracellular transport, maintenance of polarity, intracellular signal transduction, cell migration, and proliferation. During mitosis of cells, spindles are formed to perform a process in which chromosomes are arranged at the cell center and then separated to both poles. If the spindles do not function properly, cell division is inhibited and apoptosis occurs, and thus microtubules are attracting attention as targets for anticancer agents.

Drugs targeting microtubules are largely divided into two groups: drugs that stabilize microtubules and drugs that destabilize microtubules. First, microtubule stabilizers include taxane, paclitaxel (Taxol), decetaxel, and the like, and act to prevent depolymerization of microtubules and enhance polymerization. Most microtubule-stabilizing substances bind to the taxane-binding site or the overlapping site of β-tubulin. Second, microtubule destabilizers include colchicine, vinca alkaloids, and the like, which bind to the colchicine binding site or the vinca binding site. Drugs that target the microtubules themselves are effective at lower concentrations than drugs that affect microtubule polymers, and are identical in that they ultimately inhibit cell mitosis. Therefore, there is a need to develop potential tubulin polymerization inhibitors as anticancer agents.

Heat shock protein 90 (Hsp90) is one of the most abundant molecular chaperones inside eukaryotic cells and is involved in the folding, stabilization, activation, or complex formation of target proteins called 'client proteins'. Hsp90 is responsible for various biological functions related to cell growth, differentiation, and survival by regulating the stability and activity of various kinases, transcription factors, signaling molecules, enzymes, and the like under normal and stress conditions. In addition, Hsp90 contributes to the evolution of organisms and the maintenance of disease states through its role as a buffer for unstable proteins in cells. In particular, since it has become known that about 50 proteins including Her2/ErbB2, v-Src, Hif-1α, Raf-1, AKT, hTERT, and the like related to the development of cancer cells are client proteins of Hsp90, Hsp90 is receiving great attention as a target for anticancer agents. Inhibitors of Hsp90 activity induce an effect in which various oncogenic proteins are simultaneously degraded by the proteasome, and the possibility as an anticancer agent applicable to various types of cancer is being highlighted, and positive results are being shown in various stages of clinical trials currently in progress.

### DISCLOSURE OF THE INVENTION

### TECHNICAL GOALS

A technical goal to be achieved by the disclosure is to provide a chromene-benzimidazole derivative or a pharmaceutically acceptable salt thereof.

In addition, another object of the disclosure is to provide a pharmaceutical composition for preventing or treating cancer, including the chromene-benzimidazole derivative or a pharmaceutically acceptable salt thereof as an active ingredient.

However, the technical goal to be achieved by the disclosure is not limited to the above-mentioned problems, and other goals not mentioned will be clearly understood by those skilled in the art from the following description.

### TECHNICAL SOLUTIONS

In order to solve the above goals, the disclosure provides a chromene-benzimidazole derivative represented by Formula 1 or a pharmaceutically acceptable salt thereof: 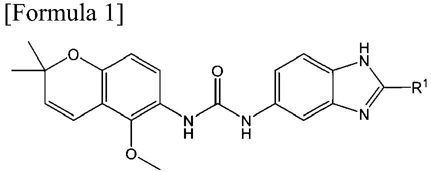 wherein in Formula 1,
R¹ is any one selected from the group consisting of hydrogen, halogen, a substituted or unsubstituted linear or branched C₁-C₆ alkyl group, a substituted or unsubstituted C₃-C₂₀ cycloalkyl group, a substituted or unsubstituted C₃-C₂₀ heterocycloalkyl group, a substituted or unsubstituted C₃-C₂₀ aryl group, and a substituted or unsubstituted C₃-C₂₀ heteroaryl group,
when the linear or branched alkyl group is substituted, the alkyl group is substituted with halogen, and
when the cycloalkyl group, heterocycloalkyl group, aryl group, or heteroaryl group is substituted, the group is substituted with at least one selected from the group consisting of linear or branched C₁-C₆ alkyl, C₁-C₁₀ cycloalkyl, halogen, hydroxy, cyano, trifluoromethyl, trifluoromethoxy, alkoxy, amino, nitro, phenyl, phenoxy, benzoyloxymethyl, indole, methylpiperazine, and benzoyl.

In an embodiment, in Formula 1,
the substituted or unsubstituted linear or branched C₁-C₆ alkyl group of R¹ is a substituted or unsubstituted methyl group, and when the methyl group is substituted, the methyl group is substituted with halogen,
the substituted or unsubstituted C₃-C₂₀ cycloalkyl group of R¹ is an unsubstituted cyclohexyl group,
the substituted or unsubstituted C₃-C₂₀ heterocycloalkyl group of R¹ is a methylpiperazine group,
the substituted or unsubstituted C₃-C₂₀ aryl group of R¹ is a substituted or unsubstituted phenyl group, and when the phenyl group is substituted, the phenyl group is substituted with at least one selected from the group consisting of linear or branched C₁-C₆ alkyl, C₁-C₁₀ cycloalkyl, halogen, hydroxy, cyano, trifluoromethyl, trifluoromethoxy, alkoxy, amino, nitro, phenyl, phenoxy, benzoyloxymethyl, indole, methylpiperazine, and benzoyl, and
the substituted or unsubstituted C₃-C₂₀ heteroaryl group of R¹ may be any one selected from the group consisting of a pyridyl group, a thiophenyl group, and a thiazolyl group.

In an embodiment, in Formula 1,
R¹ is a substituted or unsubstituted phenyl group, and
when the phenyl group is substituted, the phenyl group may be substituted with at least one selected from the group consisting of methyl, propyl, isopropyl, isobutyl, tert-butyl, cyclopropyl, cyclopentyl, cyclohexyl, adamantyl, F, Cl, hydroxy, cyano, trifluoromethyl, trifluoromethoxy, methoxy, N,N-dimethylamino, N,N-diethylamino, nitro, phenyl, phenoxy, benzoyloxymethyl, n-pentyloxy, indole, methylpiperazine, and benzoyl.

In an embodiment, the chromene-benzimidazole derivative represented by Formula 1 may be any one selected from the group consisting of the following compounds: 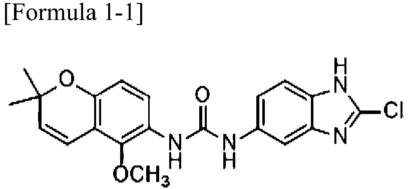 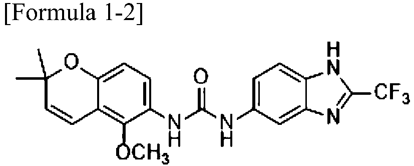 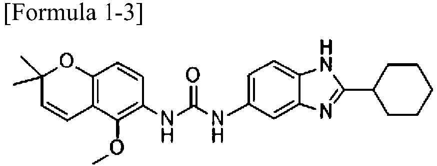 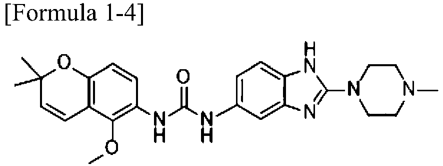 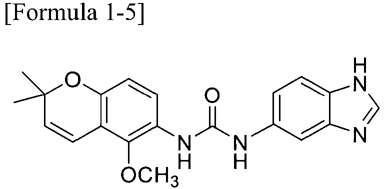 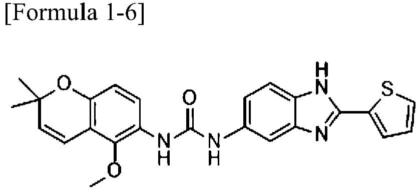 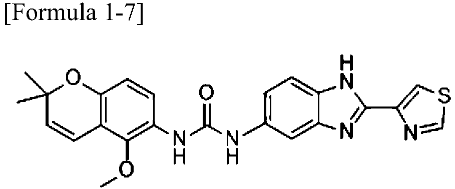 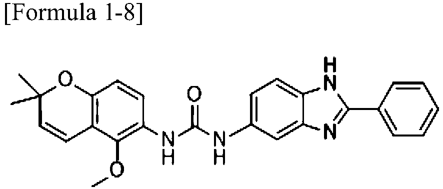 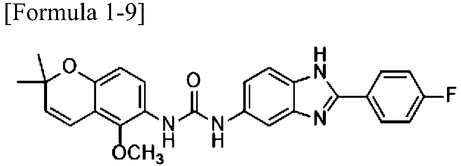 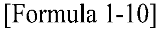 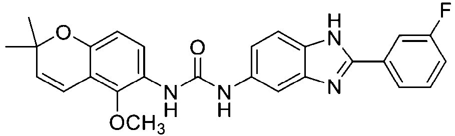 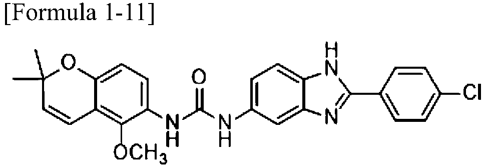 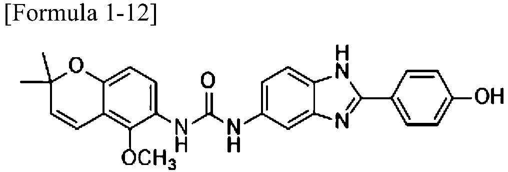 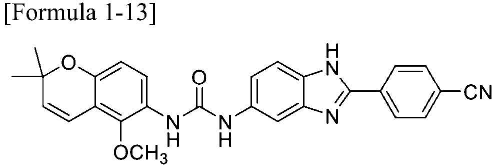 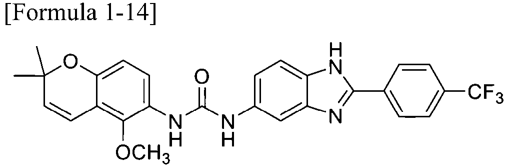 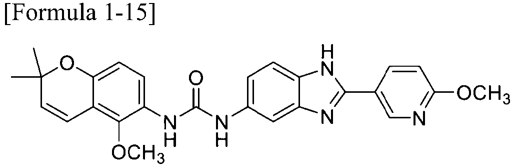 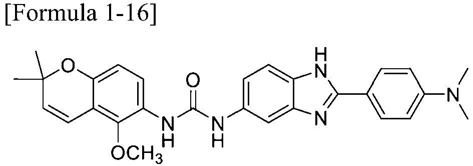 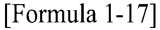 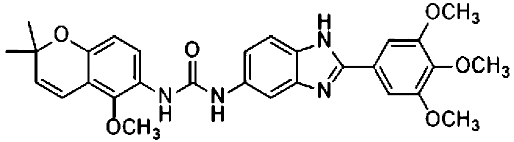 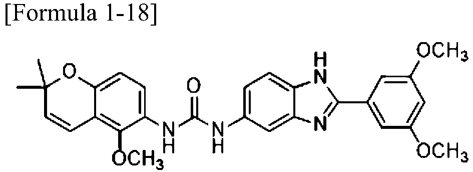 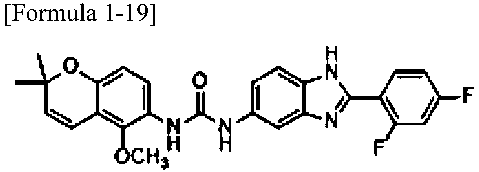 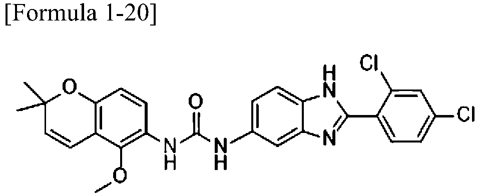 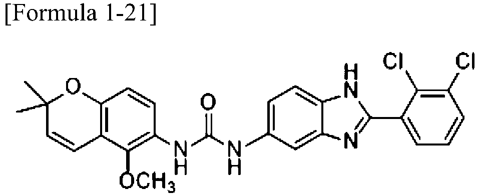 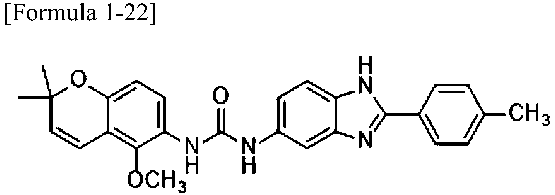 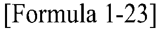 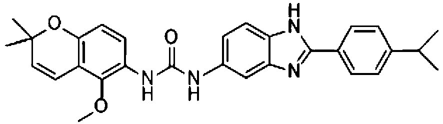 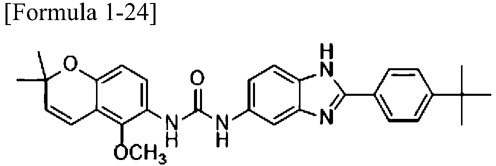 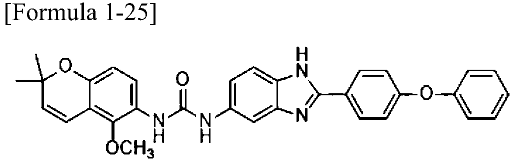 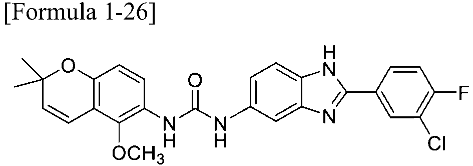 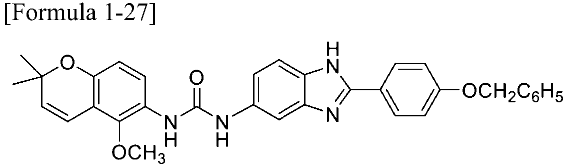 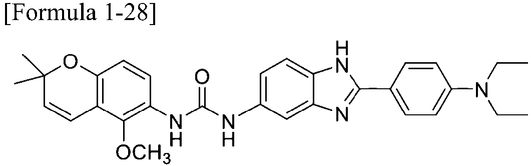 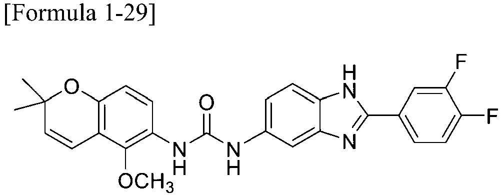 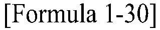 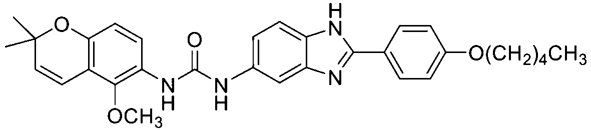 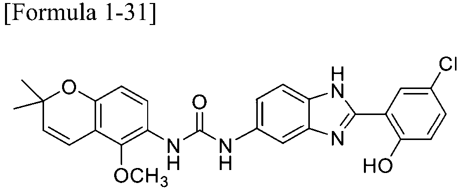 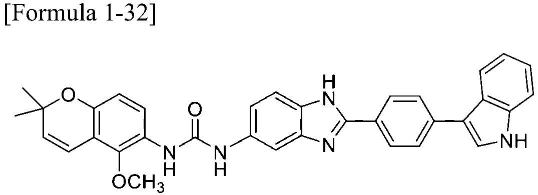 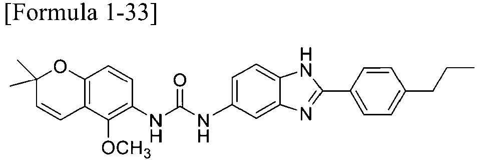 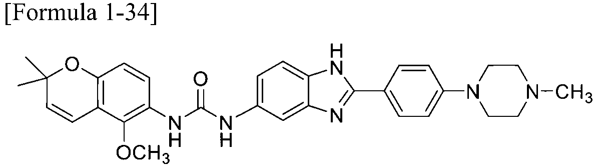 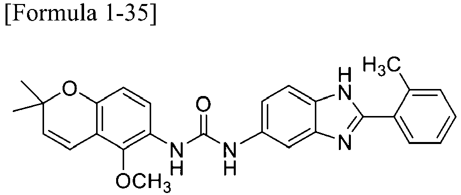 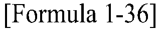 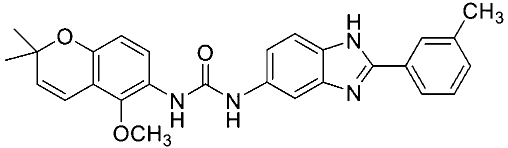 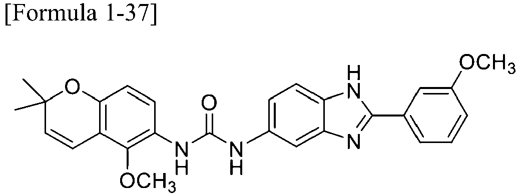 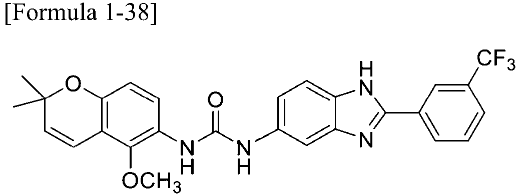 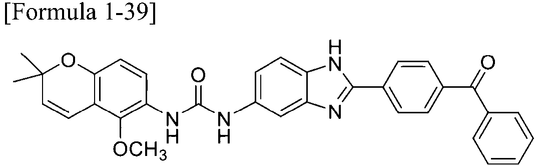 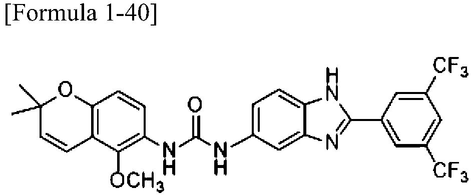 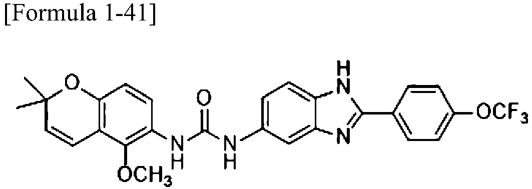 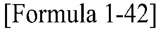 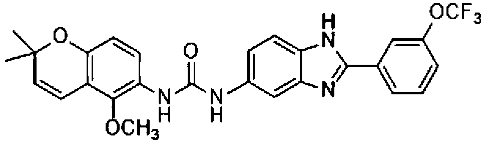 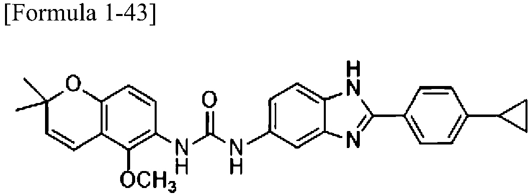 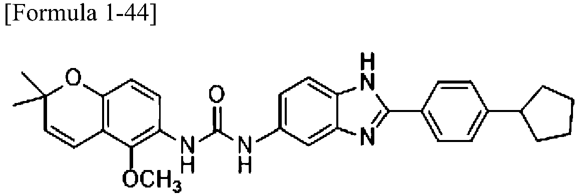 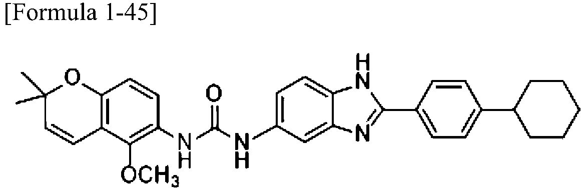 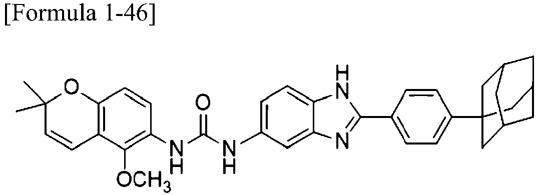 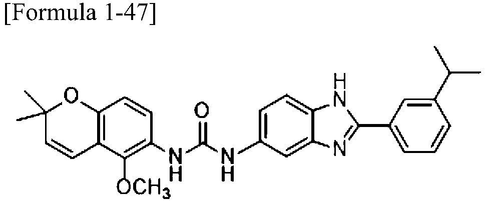 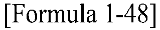 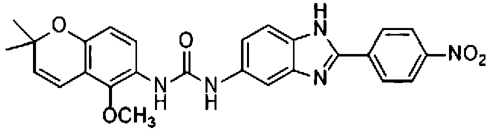 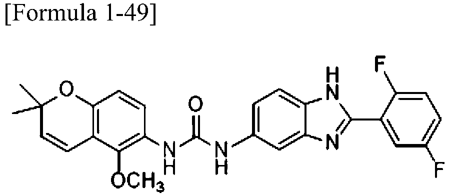 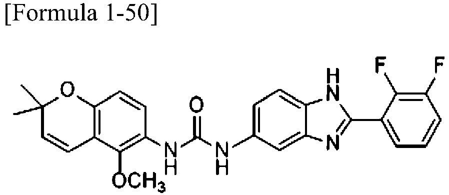 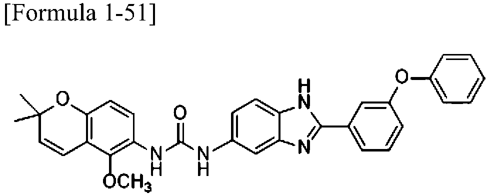 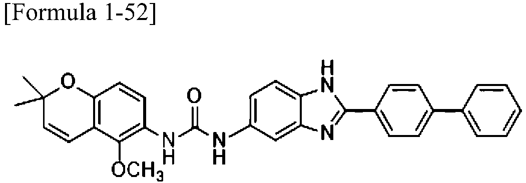 and

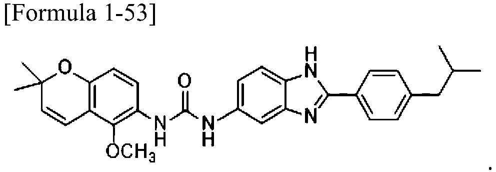

In an embodiment, the chromene-benzimidazole derivative represented by Formula 1 may be any one selected from the group consisting of the following compounds. 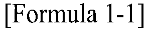 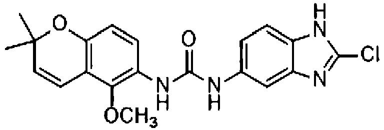 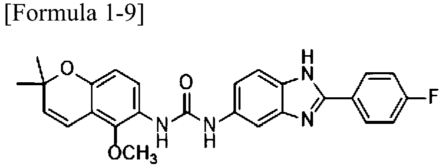 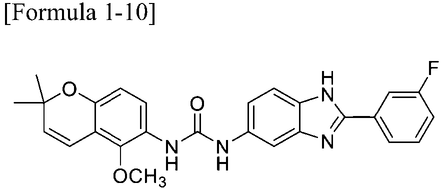 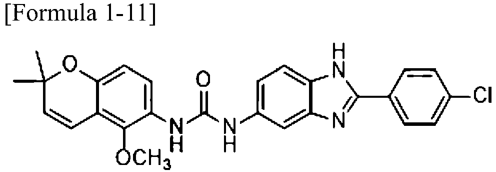 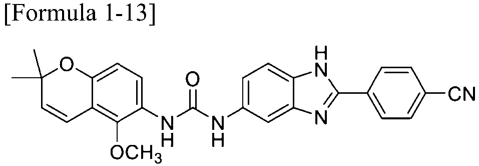 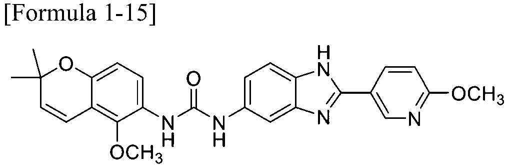 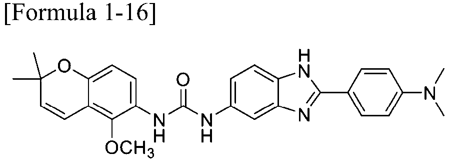 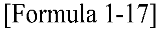 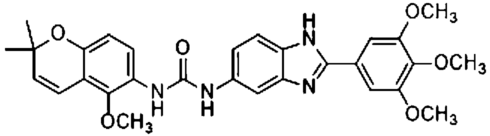 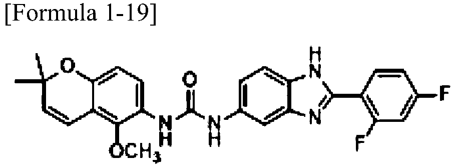 and

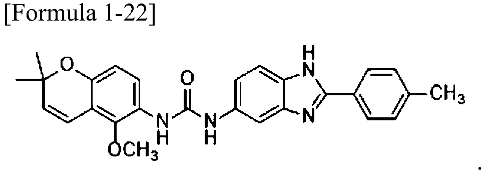

In an embodiment, the chromene-benzimidazole derivative may have both an effect of inhibiting tubulin polymerization and an effect of inhibiting a C-terminal domain of Hsp90.

In an embodiment, the pharmaceutically acceptable salt of the chromene-benzimidazole derivative may be at least one selected from the group consisting of a hydrochloride salt, a bromate salt, a sulfate salt, a phosphate salt, a nitrate salt, a citrate salt, an acetate salt, a lactate salt, a tartrate salt, a maleate salt, a gluconate salt, a succinate salt, a formate salt, a trifluoroacetate salt, an oxalate salt, a fumarate salt, a glutarate salt, an adipate salt, a methanesulfonate salt, a benzenesulfonate salt, a para-toluenesulfonate salt, a camphorsulfonate salt, a sodium salt, a potassium salt, a lithium salt, a calcium salt, and a magnesium salt.

According to another embodiment of the disclosure, there is provided a pharmaceutical composition for preventing or treating cancer, including any one of the chromene-benzimidazole derivatives, or a pharmaceutically acceptable salt thereof as an active ingredient.

In an embodiment, the cancer may be at least one selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, hematologic cancer, thymic cancer, urethral cancer, and bronchial cancer, and preferably may be triple-negative breast cancer.

In addition, the disclosure provides a composition for preventing or treating cancer, including the chromene-benzimidazole derivative or a pharmaceutically acceptable salt thereof as an active ingredient.

In addition, the disclosure provides a method of preventing or treating cancer, including administering the chromene-benzimidazole derivative or a pharmaceutically acceptable salt thereof to a subject.

In addition, the disclosure provides a use of the chromene-benzimidazole derivative or a pharmaceutically acceptable salt thereof for manufacturing a medicament for preventing or treating cancer.

In addition, the disclosure provides a method of diagnosing cancer, including administering the chromene-benzimidazole derivative or a pharmaceutically acceptable salt thereof to a subject.

In addition, the disclosure provides a use of the chromene-benzimidazole derivative or a pharmaceutically acceptable salt thereof for manufacturing a medicament for diagnosing cancer.

As an embodiment of the disclosure, the pharmaceutical composition may induce apoptosis by cell cycle arrest of cancer cells, or may induce denaturation and degradation of an oncoprotein, and preferably may perform both mechanisms of action.

As another embodiment of the disclosure, the cancer may be at least one selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, and bronchial cancer, and preferably may be breast cancer, and more preferably may be triple-negative breast cancer.

### EFFECTS OF THE INVENTION

The disclosure relates to a chromene-benzimidazole derivative or a pharmaceutically acceptable salt thereof, and a composition for preventing or treating cancer including the derivative as an active ingredient. The chromene-benzimidazole derivative of the disclosure is activated in cancer cells to inhibit tubulin polymerization and simultaneously inhibit a C-terminal domain of Hsp90 protein, thereby blocking cell cycle in cancer cells when administered to a subject, inducing apoptosis, and inhibiting activity of an oncoprotein, thereby exhibiting cytotoxicity and obtaining an anticancer effect. Therefore, the compound of the disclosure and the composition including the same may be used as a novel anticancer composition with dual targets for preventing or treating cancer, preferably for preventing or treating triple-negative breast cancer.

The effects of the disclosure are not limited to those mentioned above, and other effects not mentioned will be clearly understood by those skilled in the art from the following description.

### BRIEF DESCRIPTION OF DRAWINGS

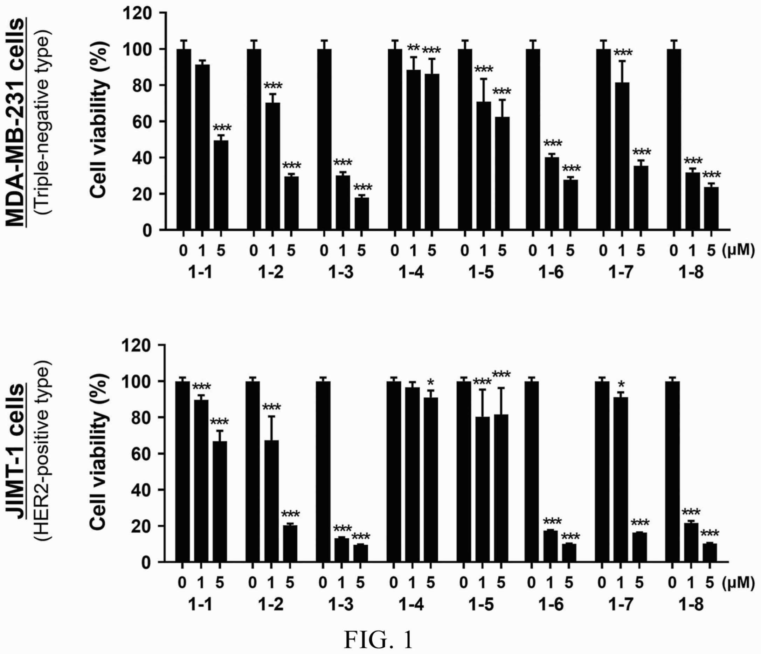

FIG. 1 shows the results of evaluating changes in cell viability through MTS assay after treating triple-negative breast cancer cell line MDA-MB-231 and HER2-positive breast cancer cell line JIMT-1 with chromene-benzimidazole derivative compounds (1-1 to 1-8) at different concentrations (0, 1, 5 µM).

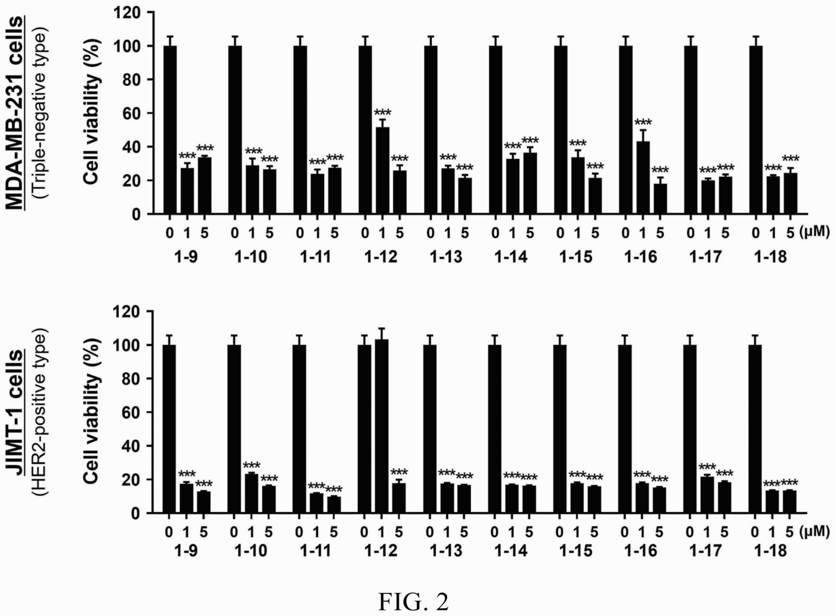

FIG. 2 shows the results of evaluating changes in cell viability through MTS assay after treating triple-negative breast cancer cell line MDA-MB-231 and HER2-positive breast cancer cell line JIMT-1 with chromene-benzimidazole derivative compounds (1-9 to 1-18) at different concentrations (0, 1, 5 µM).

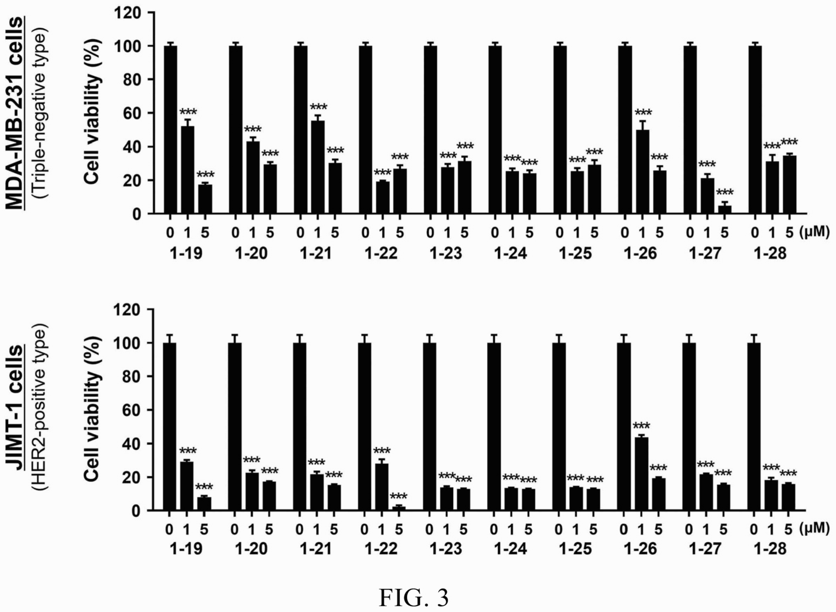

FIG. 3 shows the results of evaluating changes in cell viability through MTS assay after treating the triple-negative breast cancer cell line MDA-MB-231 and the HER2-positive breast cancer cell line JIMT-1 with chromene-benzimidazole derivative compounds (1-19 to 1-28) at different concentrations (0, 1, 5 µM).

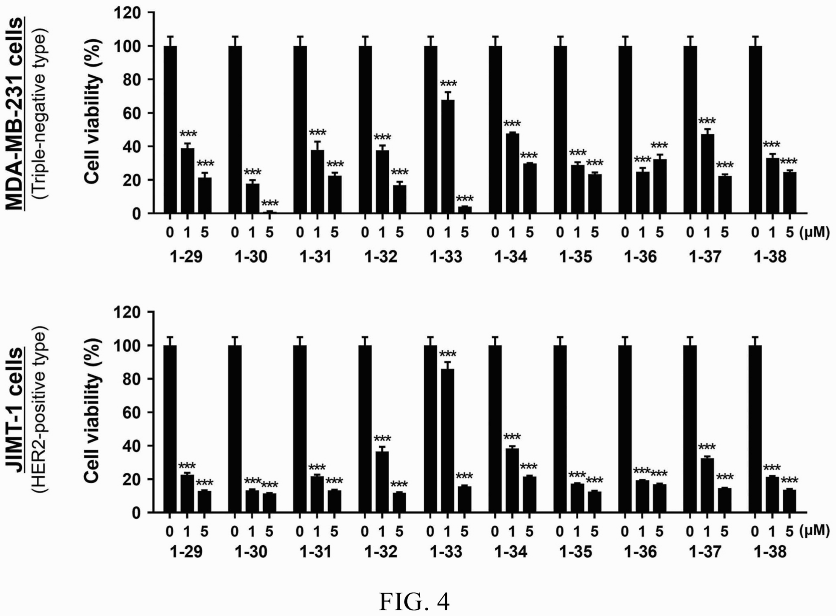

FIG. 4 shows the results of evaluating changes in cell viability through MTS assay after treating triple-negative breast cancer cell line MDA-MB-231 and HER2-positive breast cancer cell line JIMT-1 with chromene-benzimidazole derivative compounds (1-29 to 1-38) at different concentrations (0, 1, 5 µM).

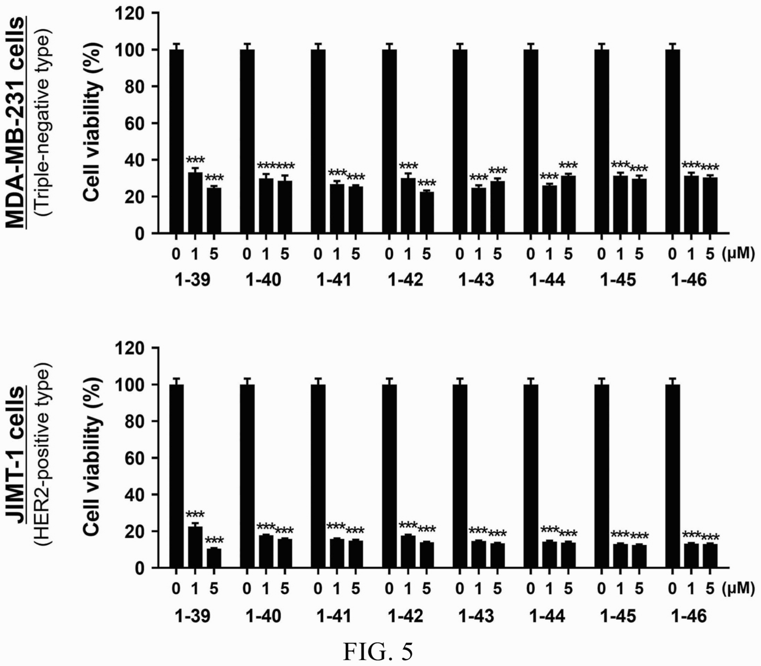

FIG. 5 shows the results of evaluating changes in cell viability through MTS assay after treating triple-negative breast cancer cell line MDA-MB-231 and HER2-positive breast cancer cell line JIMT-1 with chromene-benzimidazole derivative compounds (1-39 to 1-46) at different concentrations (0, 1, 5 µM).

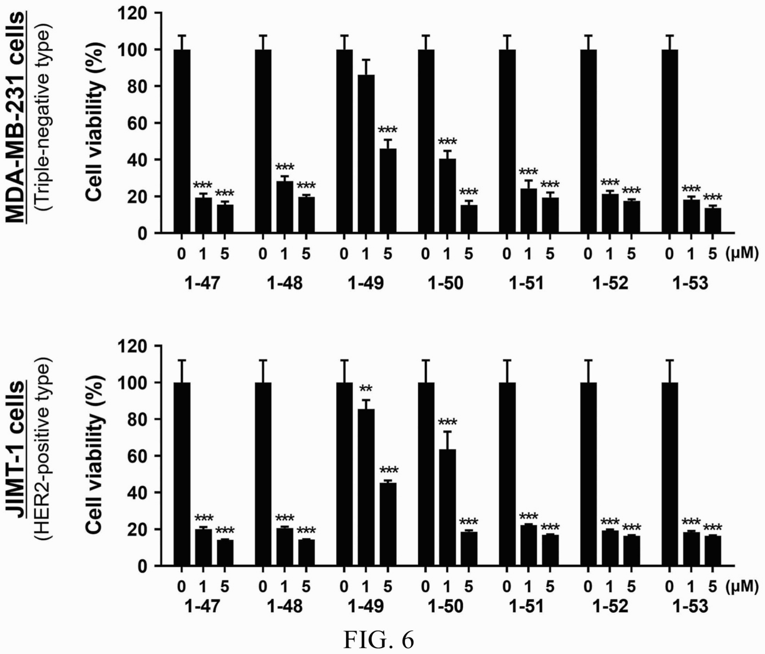

FIG. 6 shows the results of evaluating changes in cell viability through MTS assay after treating triple-negative breast cancer cell line MDA-MB-231 and HER2-positive breast cancer cell line JIMT-1 with chromene-benzimidazole derivative compounds (1-47 to 1-53) at different concentrations (0, 1, 5 µM).

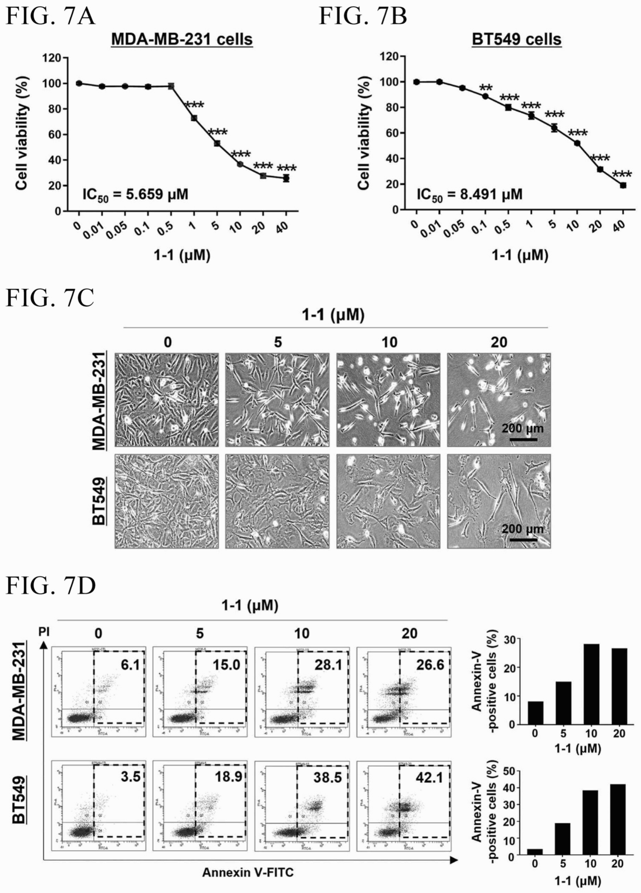

FIG. 7 confirms that chromene-benzimidazole derivative compound (1-1) inhibits cell viability in a concentration-dependent manner in triple-negative breast cancer cell lines MDA-MB-231 and BT549, induces cell death, and induces early and late apoptosis.

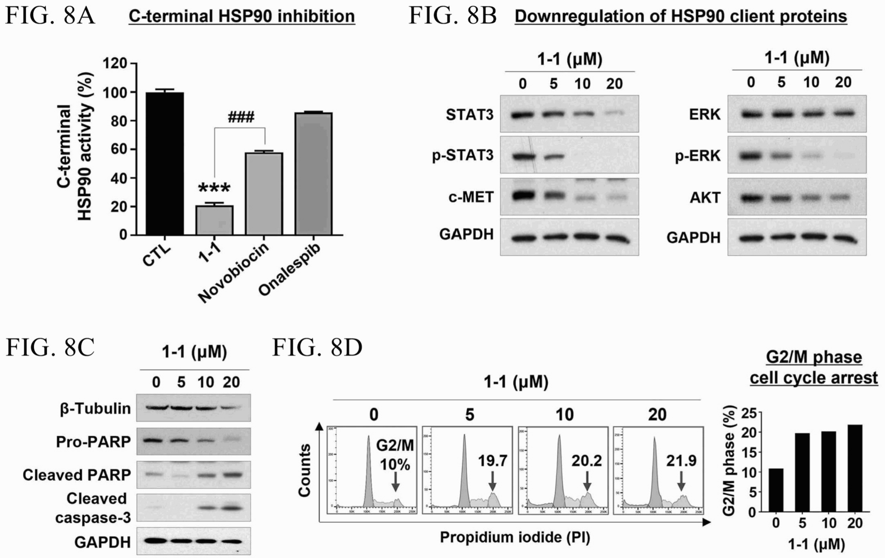

FIG. 8 confirms that the chromene-benzimidazole derivative binds to the C-terminal domain of Hsp90 to inhibit the function of Hsp90 and exerts an anticancer effect by inhibiting tubulin polymerization reaction, reduces the expression and phosphorylation of STAT3, c-MET, ERK, and AKT proteins in MDA-MB-231 and BT549 cell lines, and induces G2/M phase cell cycle arrest.

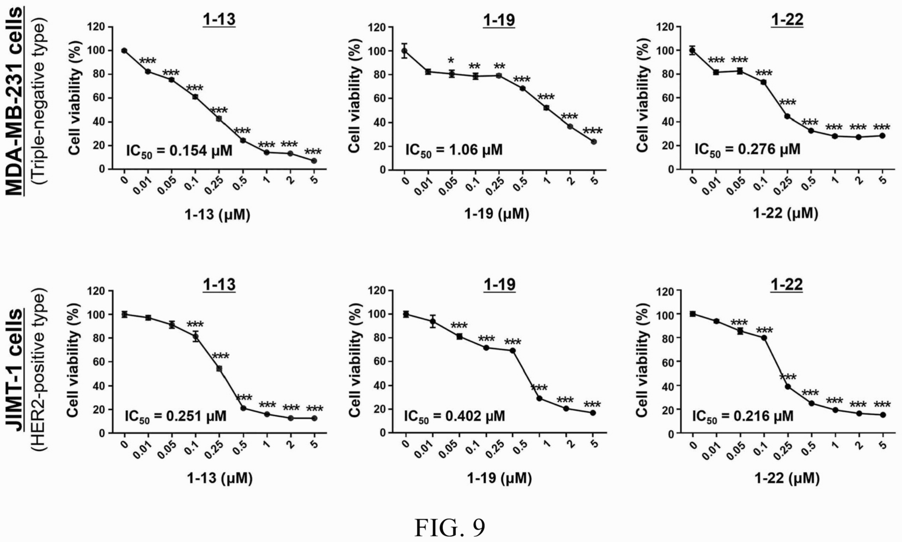

FIG. 9 shows the results of confirming concentration-dependent inhibition of cell viability and IC50 values through MTS assay after treating triple-negative breast cancer cell lines MDA-MB-231 and JIMT-1 with chromene-benzimidazole derivative compounds (1-13, 1-19, 1-22) at different concentrations.

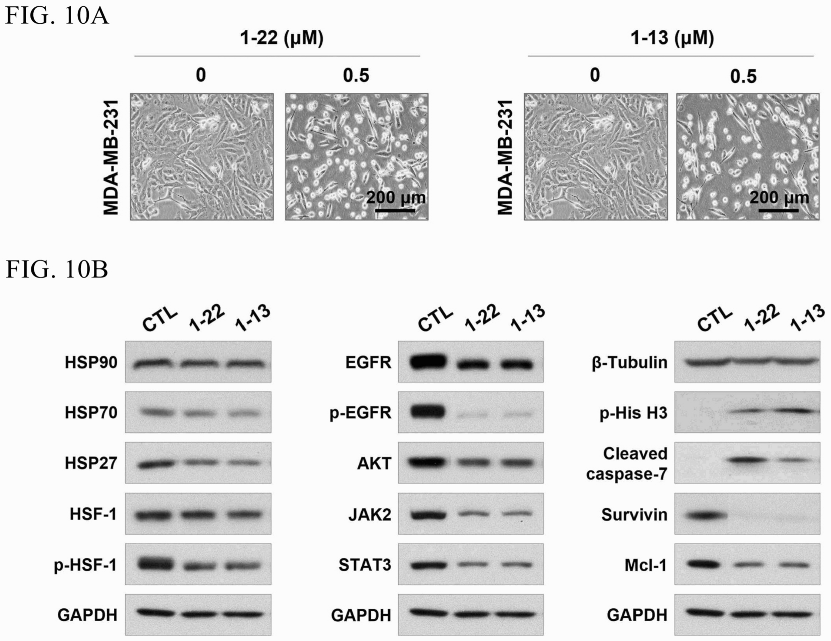

FIG. 10 shows results of observing morphological changes in cell cycle arrest and apoptosis through optical microscopy and Western blot technique after treating MDA-MB-231 cell line with compounds 1-22 and 1-13, and confirming the expression of Hsp90 complex, Hsp90 client proteins, tubulin targeting ability, and apoptosis-related proteins.

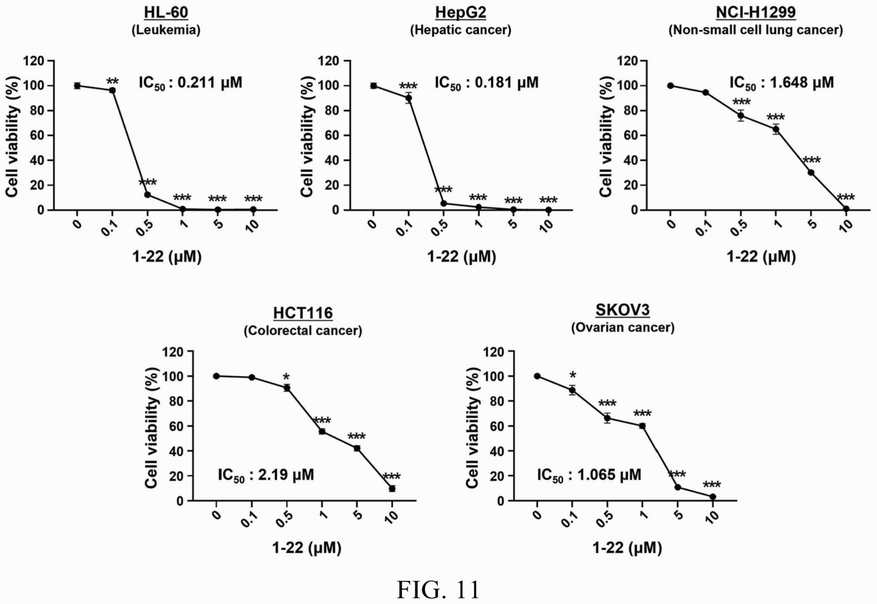

FIG. 11 confirms the effect of chromene-benzimidazole derivative compound 1-22 in hematologic cancer, liver cancer, lung cancer, colorectal cancer, and ovarian cancer cell lines.

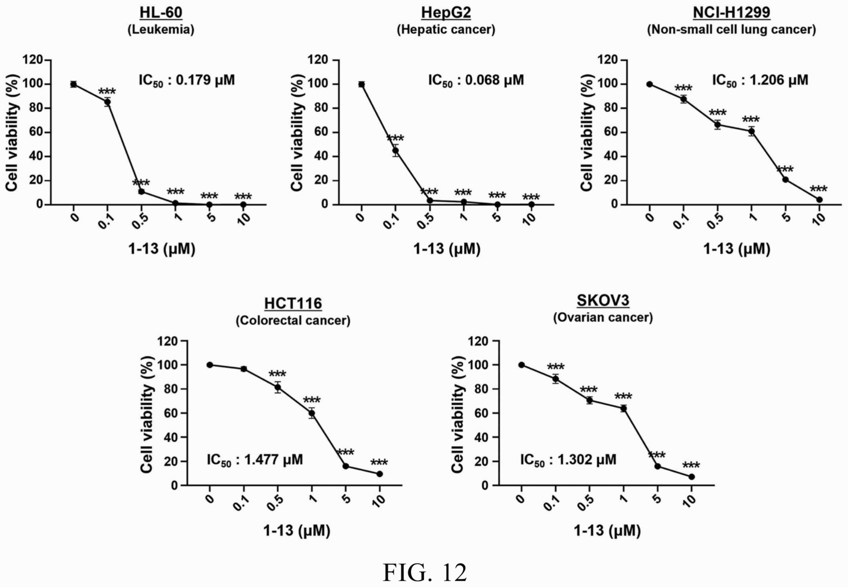

FIG. 12 confirms the effect of chromene-benzimidazole derivative compound 1-13 in hematologic cancer, liver cancer, lung cancer, colorectal cancer, and ovarian cancer cell lines.

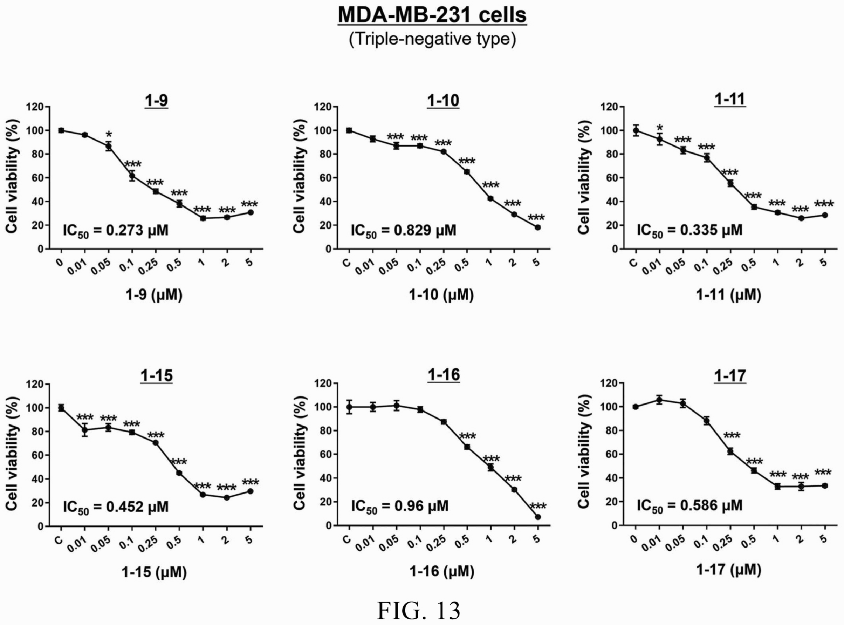

FIG. 13 shows the results of confirming concentration-dependent cell viability (MTS assay) inhibitory effect and confirming IC50 values by treating the triple-negative breast cancer cell line MDA-MB-231 with chromene-benzimidazole derivative compounds (1-9, 1-10, 1-11, 1-15, 1-16, and 1-17).

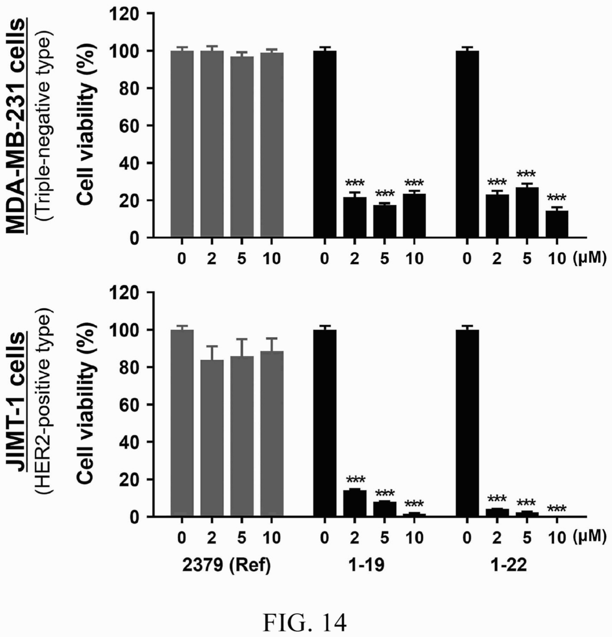

FIG. 14 shows the results of comparatively evaluating cell viability of a reference compound having a structure similar to chromene-benzimidazole derivative compounds (1-19 and 1-22) in MDA-MB-231 and JIMT-1 cell lines.

### BEST MODE FOR CARRYING OUT THE INVENTION

The present inventors have confirmed that benzimidazole derivatives have anticancer effects by inhibiting tubulin synthesis, thereby causing cell cycle arrest and thereby inhibiting cancer cell division, and that chromene derivatives can have anticancer effects by inhibiting the C-terminal domain of Hsp90. They have also confirmed the possibility of activating both mechanisms of action by combining benzimidazole and chromene in a single compound molecule, and thus, they have completed the invention and provide a chromene-benzimidazole derivative or a pharmaceutically acceptable salt thereof as a novel dual-target anticancer drug.

From the above results, the disclosure provides a chromene-benzimidazole derivative represented by [Formula 1] or a pharmaceutically acceptable salt thereof:

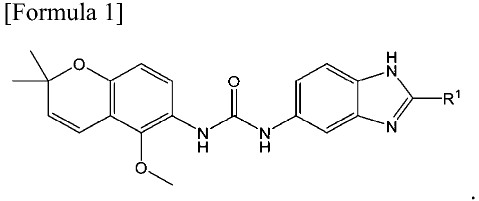

In Formula 1,
R¹ is any one selected from the group consisting of hydrogen, halogen, a substituted or unsubstituted linear or branched C₁-C₆ alkyl group, a substituted or unsubstituted C₃-C₂₀ cycloalkyl group, a substituted or unsubstituted C₃-C₂₀ heterocycloalkyl group, a substituted or unsubstituted C₃-C₂₀ aryl group, and a substituted or unsubstituted C₃-C₂₀ heteroaryl group,
when the linear or branched alkyl group is substituted, the alkyl group is substituted with halogen, and
when the cycloalkyl group, heterocycloalkyl group, aryl group, or heteroaryl group is substituted, the group is substituted with at least one selected from the group consisting of linear or branched C₁-C₆ alkyl, C₁-C₁₀ cycloalkyl, halogen, hydroxy, cyano, trifluoromethyl, trifluoromethoxy, alkoxy, amino, nitro, phenyl, phenoxy, benzoyloxymethyl, indole, methylpiperazine, and benzoyl.

In the disclosure, the term "substitution" refers to a reaction in which an atom or atomic group included in a molecule of a compound is replaced with another atom or atomic group.

In the disclosure, the term "chain" refers to a molecule having a chain structure, and the chain structure is a chemical structure in which carbon atoms are linked in a chain shape, and there are straight chain shapes and branched shapes.

In the disclosure, the term "cyclic" refers to a structure in which both ends of the skeleton of an organic compound are connected to form a ring shape.

In the disclosure, the term "linear or branched alkyl group" means a monovalent linear or branched hydrocarbon residue consisting only of carbon and hydrogen atoms, having 1 to 6 carbon atoms.

In the disclosure, the term "cyclic alkyl group" or "cycloalkyl group" means a cyclic saturated hydrocarbon residue.

Examples of the alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl, and the like.

In the disclosure, the term "heterocycloalkyl group" typically refers to a saturated or unsaturated (but not aromatic) cyclohydrocarbon, which may be optionally unsubstituted, monosubstituted or polysubstituted, and in the structure of which at least one is selected from a heteroatom of N, O or S.

In the disclosure, the term "aryl group" means an unsaturated aromatic ring compound having 3 to 12 carbon atoms having a single ring (e.g., phenyl) or multiple condensed rings (e.g., naphthyl). Examples of such aryl groups include, but are not limited to, phenyl, naphthyl, and the like.

In the disclosure, the term "heteroaryl group" refers to a single ring or multiple condensed rings in which at least one of the atoms constituting the ring has a heteroatom of N, O or S. Examples of such heteroaryl groups include, but are not limited to, a pyridyl group, a pyrimidinyl group, a pyrazinyl group, an oxazolyl group, and a furyl group.

In the disclosure, the term "alkoxy group" means an alkyl group (-O-R) bonded to oxygen. Examples of such alkoxy groups include, but are not limited to, methoxy groups, ethoxy groups, propoxy groups, and butoxy groups.

In the disclosure, the "halogen group" may be fluorine (F), chloride (Cl), bromine (Br), or iodine (I).

As a preferred embodiment of the disclosure, the compound represented by Formula 1 is preferably at least one selected from the group consisting of the following compounds: 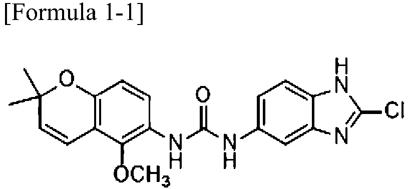 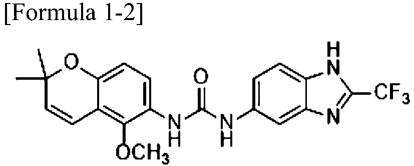 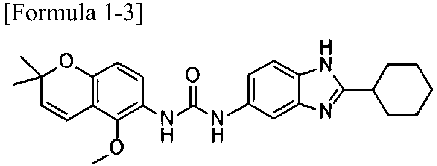 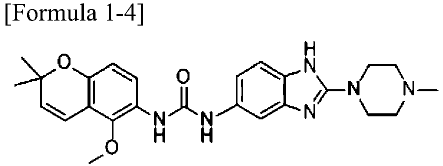 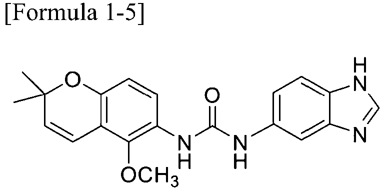 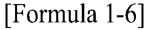 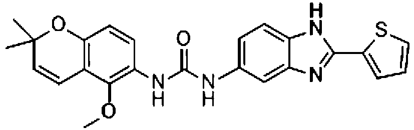 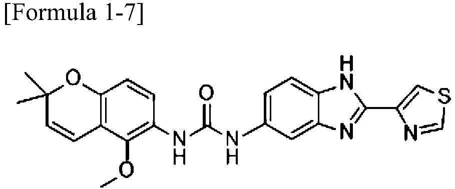 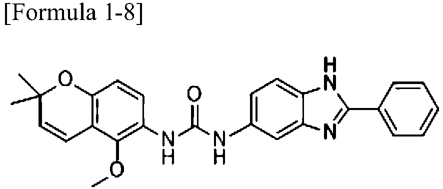 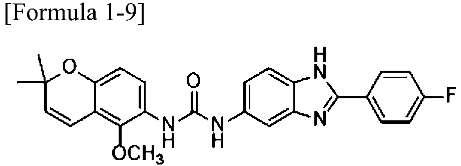 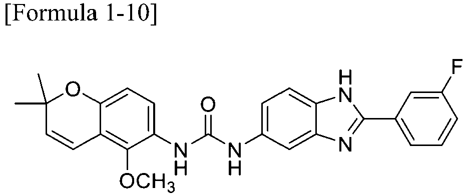 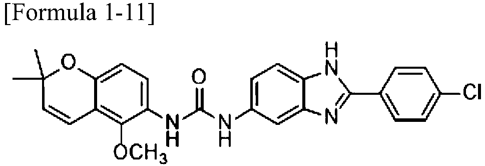 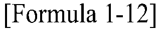 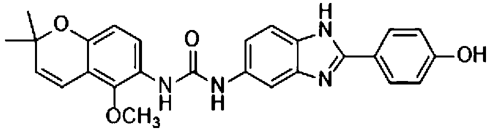 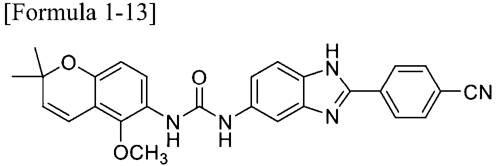 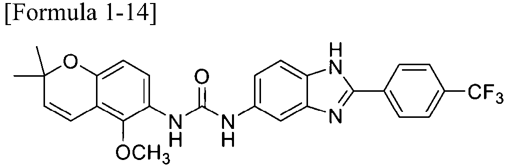 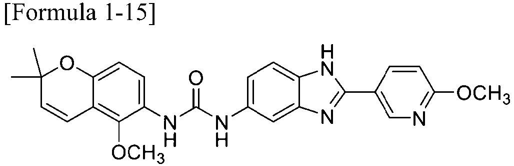 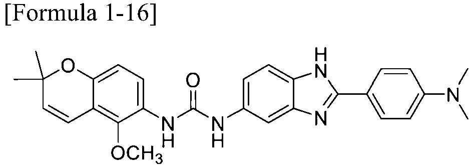 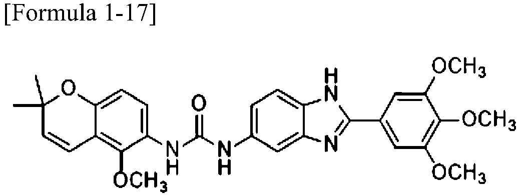 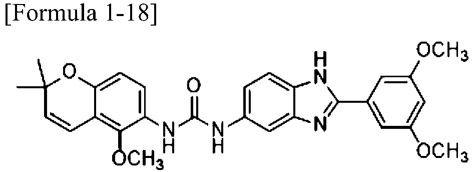 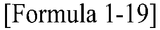 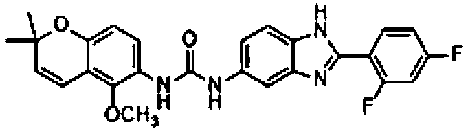 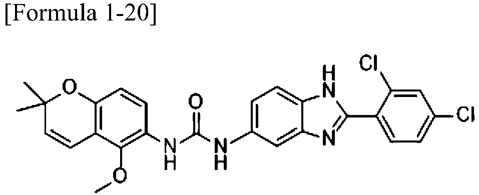 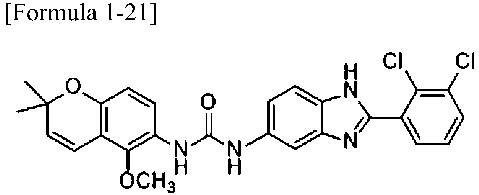 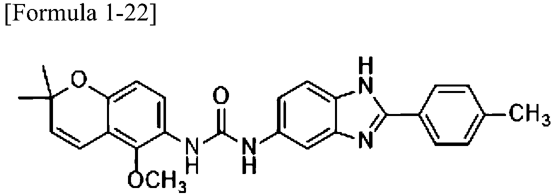 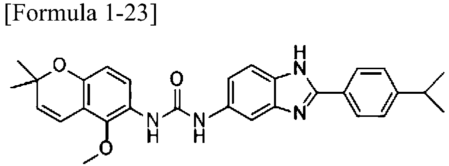 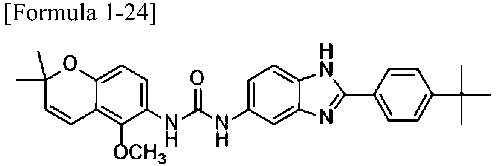 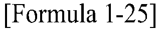 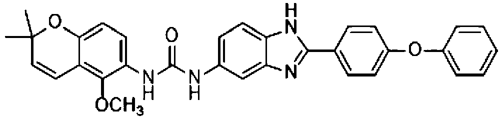 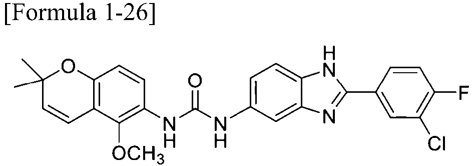 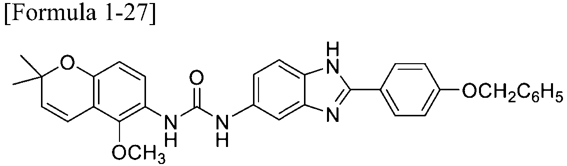 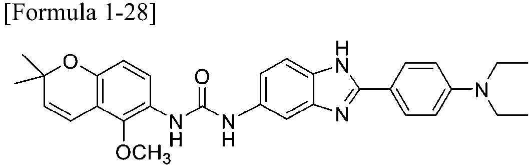 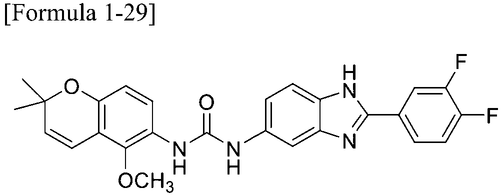 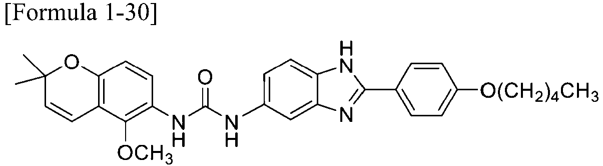 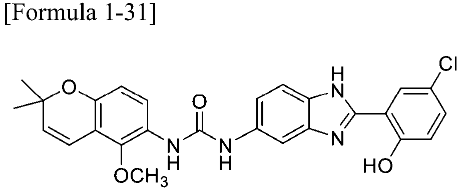 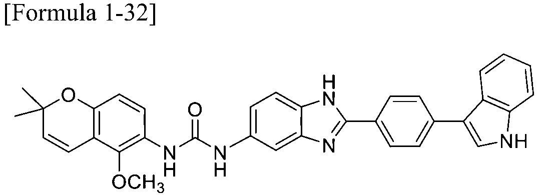 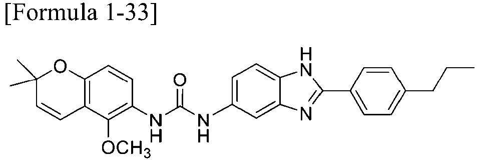 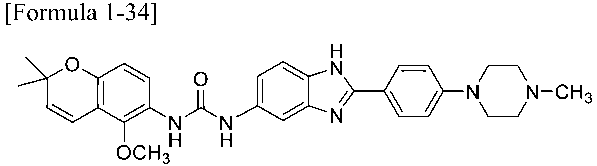 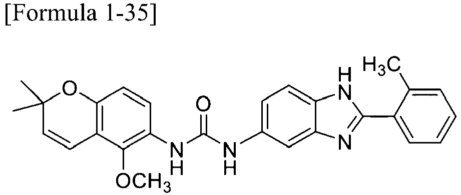 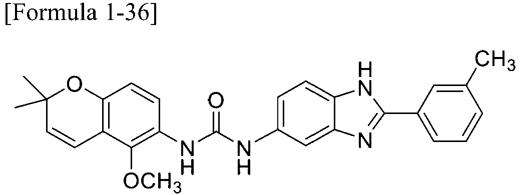 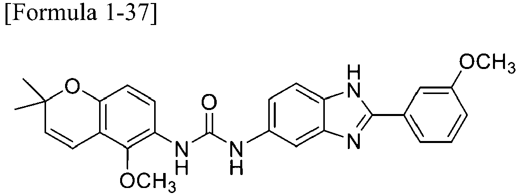 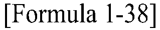 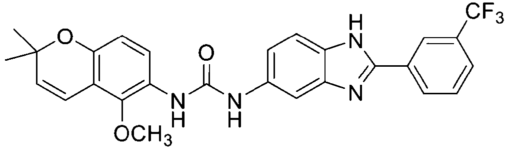 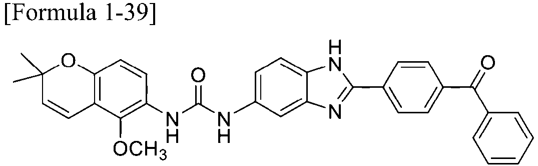 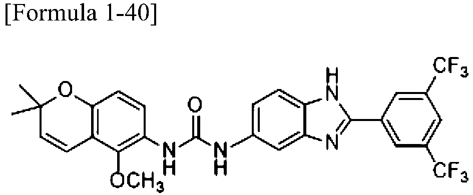 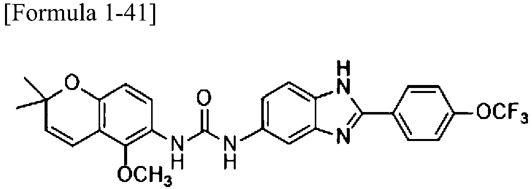 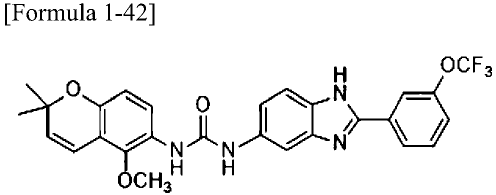 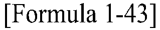 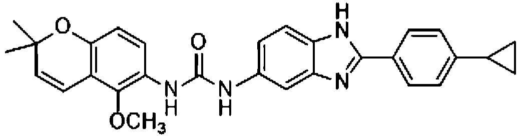 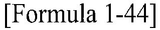 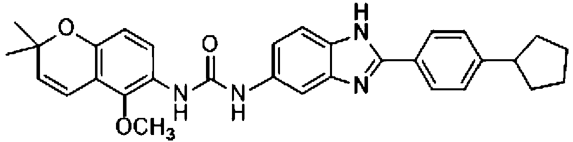 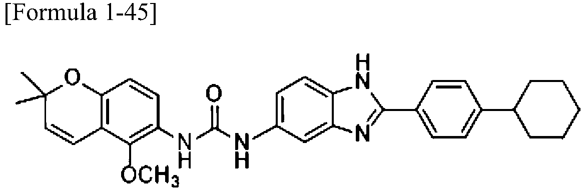 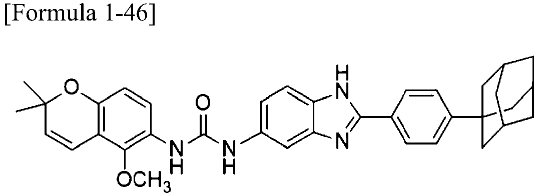 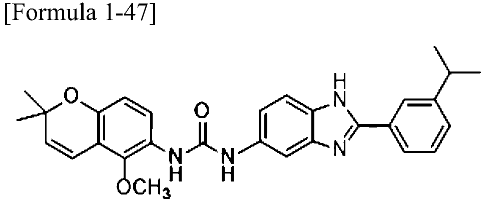 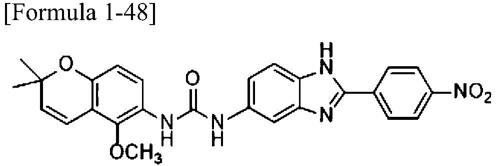 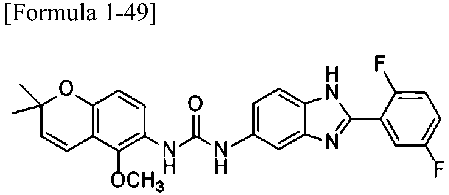 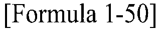 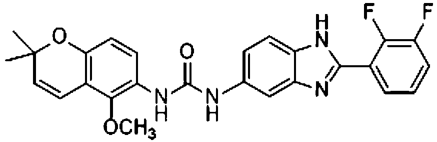 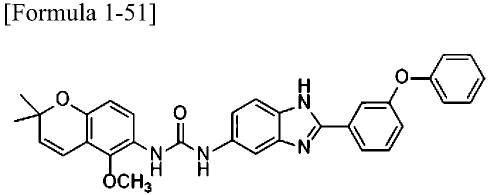 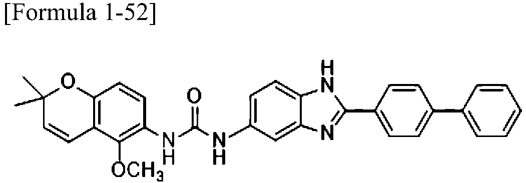 and 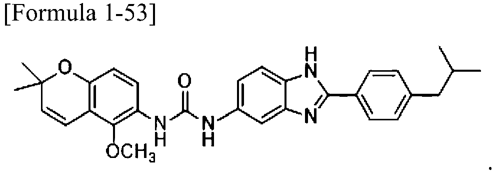 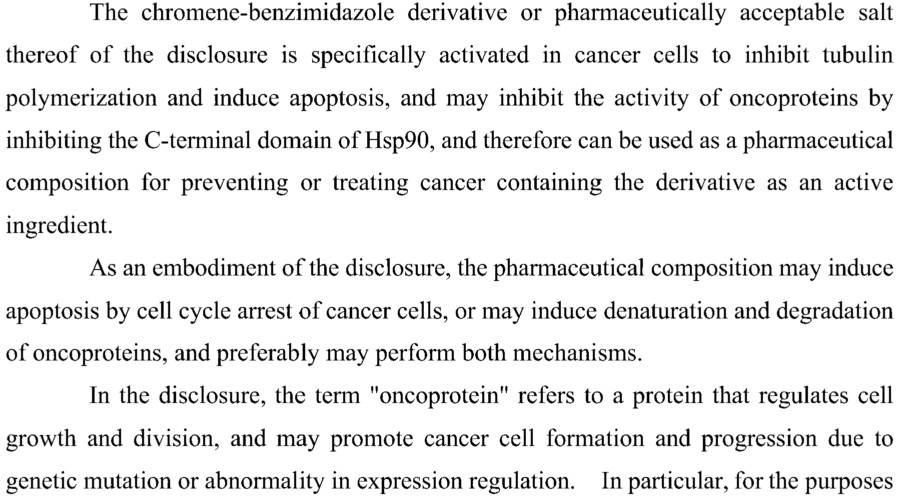 of the disclosure, the oncoprotein may be a client protein of Hsp90, preferably Her2/ErbB2, v-Src, Hif-1α, Raf-1, AKT, hTERT, ERK, p-ERK, STAT3, c-MET, and the like.

In the disclosure, the term "pharmaceutically acceptable salt" means a formulation of a compound that does not cause serious irritation to an organism to which the compound is administered and does not impair the biological activity and physical properties of the compound. The pharmaceutically acceptable salt may be obtained by reacting the compound of the disclosure with an inorganic acid such as hydrochloric acid, hydrobromic acid, sulfuric acid, nitric acid, or phosphoric acid; a sulfonic acid such as methanesulfonic acid, ethanesulfonic acid, or p-toluenesulfonic acid; or an organic carboxylic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, or salicylic acid. In addition, the pharmaceutically acceptable salt may be obtained by reacting the compound of the disclosure with a base to form a salt, including salts such as ammonium salts; alkali metal salts such as sodium or potassium salts; alkaline earth metal salts such as calcium or magnesium salts; salts of organic bases such as dicyclohexylamine, N-methyl-D-glucamine, or tris(hydroxymethyl)methylamine; and amino acid salts such as arginine or lysine.

Furthermore, the chromene-benzimidazole derivative or the pharmaceutically acceptable salt thereof may include not only pharmaceutically acceptable salts but also all salts, hydrates, and solvates capable of being prepared by conventional methods.

In addition, the disclosure may provide a method of preventing, treating, and/or diagnosing cancer, including administering the chromene-benzimidazole derivative or a pharmaceutically acceptable salt thereof to a subject.

In the disclosure, the term "prevention" means any act of inhibiting or delaying the occurrence, spread or recurrence of the cancer by administering the composition of the disclosure, and "treatment" means any act of improving or beneficially changing the symptoms of the disease by administering the composition of the disclosure.

In the disclosure, the term "pharmaceutical composition" means a composition manufactured for the purpose of preventing or treating the above-described disease, and can be formulated and used in various forms according to conventional methods. For example, the pharmaceutical composition can be formulated into oral dosage forms such as powder, granules, tablets, capsules, suspensions, emulsions, and syrups, and can be formulated and used in the form of external preparations, suppositories, and sterile injectable solutions.

In the disclosure, "including as an active ingredient" means that the corresponding component is included in an amount necessary or sufficient to realize a desired biological effect. In actual application, the determination of the amount included as an active ingredient is an amount for treating a target disease, and may be determined by considering factors that do not cause other toxicity, and may vary according to various factors such as, for example, the disease or condition being treated, the form of the composition being administered, the size of the subject, or the severity of the disease or condition. A person skilled in the art to which the disclosure pertains can empirically determine the effective amount of an individual composition without undue experimentation.

In addition, the pharmaceutical composition of the disclosure may, depending on each formulation, additionally contain one or more pharmaceutically acceptable carriers in addition to the active ingredients described above.

The pharmaceutically acceptable carrier may be saline solution, sterile water, Ringer's solution, buffered saline, dextrose solution, maltodextrin solution, glycerol, ethanol, and mixtures of one or more of these components, and may further include other conventional additives such as antioxidants, buffers, and bacteriostatic agents, as needed. In addition, diluents, dispersants, surfactants, binders and lubricants may be additionally added to formulate the composition into injectable formulations such as aqueous solutions, suspensions and emulsions, pills, capsules, granules or tablets. Furthermore, the composition may be preferably formulated for each disease or ingredient using an appropriate method in the art or a method disclosed in Remington's Pharmaceutical Science (Mack Publishing Company, Easton PA).

The composition of the disclosure may be administered orally or parenterally in a pharmaceutically effective amount according to a desired method, and the term "pharmaceutically effective amount" of the disclosure means an amount that is sufficient to treat a disease with a reasonable benefit/risk ratio applicable to medical treatment and does not cause side effects, and the effective dose level may be determined according to factors including the patient's health condition, severity, activity of the drug, sensitivity to the drug, administration method, administration time, administration route and excretion rate, treatment period, drugs used in combination or concurrently, and other factors well known in the medical field.

Accordingly, the pharmaceutical composition of the disclosure may be administered to a subject to prevent, treat, and/or diagnose cancer, and the cancer may be skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, or bronchial cancer, but is not limited thereto, and preferably, may be a cancer having a characteristic in that acidity is higher than that of normal cells and cytotoxicity is inhibited by a tubulin polymerization inhibitor, and non-limiting examples thereof include breast cancer, preferably triple-negative breast cancer.

In the disclosure, the term "subject" is not limited to a mammal such as livestock or a human that requires prevention, treatment, and/or diagnosis of the cancer, but may preferably be a human.

The pharmaceutical composition of the disclosure may be formulated in various forms for administration to a subject, and a representative form for parenteral administration is an injectable form, preferably an isotonic aqueous solution or suspension. Injectable formulations can be prepared according to techniques known in the art using suitable dispersing or wetting agents and suspending agents. For example, each component can be dissolved in saline or a buffer solution and formulated for injection. In addition, formulations for oral administration include, for example, ingestible tablets, buccal tablets, troches, capsules, elixirs, suspensions, syrups, and wafers, and these formulations may contain, in addition to active ingredients, diluents (e.g., lactose, dextrose, sucrose, mannitol, sorbitol, cellulose and/or glycine) and lubricants (e.g., silica, talc, stearic acid and its magnesium or calcium salts and/or polyethylene glycol). The tablets may contain binding agents such as magnesium aluminum silicate, starch paste, gelatin, tragacanth, methylcellulose, sodium carboxymethylcellulose and/or polyvinylpyrrolidine, and may optionally further contain disintegrants such as starch, agar, alginic acid or its sodium salt, absorbents, colorants, flavoring agents and/or sweetening agents. The formulations may be prepared by conventional mixing, granulation or coating methods.

In addition, the pharmaceutical composition of the disclosure may further include auxiliary agents such as preservatives, wetting agents, emulsifying agents, salts for osmotic pressure control or buffers, and other therapeutically useful substances, and may be formulated according to conventional methods.

The pharmaceutical composition according to the disclosure may be administered through various routes including oral, transdermal, subcutaneous, intravenous or intramuscular routes, and the dosage of the active ingredient may be appropriately selected according to various factors such as the route of administration, the patient's age, sex, weight, and the severity of the patient's condition. In addition, the composition of the disclosure may be administered in combination with known compounds that can enhance the desired effect.

As routes of administration for the pharmaceutical composition according to the disclosure, the pharmaceutical composition may be administered to humans and animals orally or parenterally such as intravenously, subcutaneously, intranasally, or intraperitoneally. Oral administration also includes sublingual application. Parenteral administration includes injection methods such as subcutaneous injection, intramuscular injection, and intravenous injection, and instillation methods.

In the pharmaceutical composition of the disclosure, the total effective amount of the chromene-benzimidazole derivative or a pharmaceutically acceptable salt thereof according to the disclosure may be administered to a patient as a single dose, or may be administered by a fractionated treatment protocol in which multiple doses are administered over a long period of time. The pharmaceutical composition of the disclosure may vary the content of the active ingredient depending on the severity of the disease, but may typically be administered several times a day at an effective dose of 100 *µ*g to 3,000 mg per administration for adults. However, regarding the concentration of the chromene-benzimidazole derivative or the pharmaceutically acceptable salt thereof, an effective dosage for a patient may be determined by considering various factors such as age, body weight, health condition, sex, severity of the disease, diet, and excretion rate of a patient, as well as a route of administration and a frequency of treatment of the drug.

In addition, the pharmaceutical composition according to the disclosure is not particularly limited in its formulation, administration route, and administration method as long as it exhibits the effects of the disclosure, and the pharmaceutical composition of the disclosure may additionally include a known anticancer agent in addition to the chromene-benzimidazole derivative or a pharmaceutically acceptable salt thereof as an active ingredient, and may be used in combination with other known treatments for the treatment of these diseases.

The terminology used herein is for the purpose of describing particular embodiments only and is not to be limiting of the embodiments. The singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the technical scope of the disclosure.

When describing the embodiments with reference to the accompanying drawings, like reference numerals refer to like components and a repeated description related thereto will be omitted. In the description of embodiments, detailed description of well-known related technology will be omitted when it is deemed that such description will cause ambiguous interpretation of the disclosure.

### Best Mode for Carrying Out the Invention

### [Examples]

### Example 1. Preparation of Chromene-Benzimidazole Derivatives

All chemical reagents used were commercially available. ¹H NMR spectra were recorded on a Bruker Avance III 400 MHz, with TMS used as an internal standard.

### 1.1 Synthesis of Formula 1-1 (1-(2-chloro-1H-benzo[d]imidazol-5-yl)-3-(5-methoxy-2,2-dimethyl-2H-chromen-6-yl)urea)

The chromene-benzimidazole derivative of Formula 1-1 was synthesized by the following two methods.

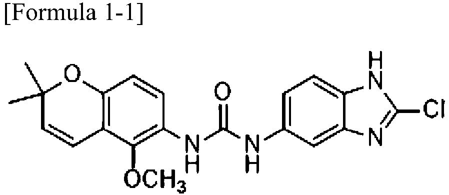

### Method 1

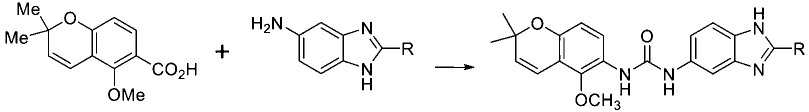

To a stirred solution of 5-Methoxy-2,2-dimethyl-2H-chromene-6-carboxylic acid (47 mg, 0.2 mmol) and (PhO)₂P(O)N₃ (DPPA, 46.0 mg, 0.17 mmol) in benzene (2 mL) was added Et₃N (0.05 ml, 0.5 mmol) dropwise at room temperature. The reaction mixture was stirred at room temperature for 3 hours, then at 80°C for 6 hours, and the solvent was removed to obtain the desired residue (crude isocyanate). To the residue, dried toluene (2 ml) was added dropwise and stirred to prepare a solution, which was then slowly added dropwise to a THF (3 ml) solution of 2-chloro-1H-benzo[d]imidazol-5-amine (36.8 mg, 0.22 mmol) as an amine (free amine) and Et₃N (0.028 ml, 0.2 mmol). The reaction mixture was stirred at room temperature for 12 hours, and the solvent was removed to obtain the target residue, which was purified by column chromatography (SiO₂, MC:MeOH, 20/1, v/v) to obtain the target compound of Formula 1-1 (51 mg, 0.188 mmol) as a white solid.

Yield 64%
¹H NMR(400 MHz, DMSO-*d*₆) δ 12.9(s, br, 1H), 9.19 (s, 1H), 8.07 (s, 1H), 7.87(s, 1H), 7.82 (d, *J* = 8.6Hz, 1H), 7.42 (d, *J* = 8.6 Hz, 1H), 7.05 (s, 1H), 6.56 (d, *J* = 9.9 Hz, 1H), 6.52(d, J = 9.9 Hz, 1H), 5.82 (d, *J* = 9.9 Hz, 1H), 3.73 (s, 3H), 1.37 (s, 6H).

### Method 2

5-Methoxy-2,2-dimethyl-2H-chromene-6-carboxylic acid (0.117 g, 0.5 mmol) was dissolved in DMF (2 mL), and then 2-chloro-1H-benzo[d]imidazol-5-amine (0.0838 g, 0.5 mmol), HBTU (1-[Bis(dimethylamino)methylene]-1H-benzotriazolium 3-Oxide Hexafluorophosphate, 0.22 g), and N,N-Diisopropylethylamine (DIEA, 0.4 mL) were added, respectively, and the mixture was reacted at 100°C for 2 hours in a microwave reactor. This reaction mixture solution was diluted with an excess of ethyl acetate, extracted, washed with cold water, and the solvent was removed. The oily reaction mixture was separated by silica gel column chromatography using EtOAC:Hexane, (9/1) to obtain the compound of Formula 1-1 (0.045 g) as a light brown solid.

Yield 50%
¹H NMR(400 MHz, DMSO-*d*₆) δ 10.59(s, 1H), 10.00(s, 1H), 7.59(s, 1H), 7.39(d, J=8.4Hz, 1H), 7.15(d, J=8.4Hz, 1H), 6.86(d, J=8.0Hz, 1H), 6.64(m, 2H), 5.88(d, J=10.0Hz, 1H), 3.76(s, 3H), 1.41(s, 6H)

### 1.2 Synthesis of Formula 1-2 (1-(5-methoxy-2,2-dimethyl-2H-chromen-6-yl)-3-(2-(trifluoromethyl)-1H-benzo[d]imidazol-5-yl)urea)

The chromene-benzimidazole derivative of Formula 1-2 was synthesized as follows.

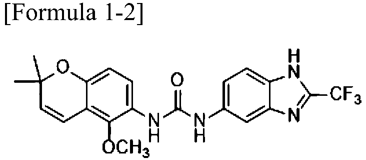

5-Methoxy-2,2-dimethyl-2H-chromene-6-carboxylic acid and 2-(trifluoromethyl)-1H-1,3-benzodiazol-5-amine were reacted in a similar manner as in Method 1 of Example 1-1 to obtain Compound 1-2.

Yield 65%
¹H NMR (400 MHz, DMSO-*d*₆) δ 13.68(bs, 1H), 9.37(bs, 1H), 8.14(s, 2H), 7.85(d, J=8.8Hz, 1H), 7.68(m, 1H), 7.10(m, 1H), 6.55(m, 2H), 5.84(d, J=9.6Hz, 1H), 3.74(s, 3H), 1.37(s, 6H)

### 1.3 Synthesis of Formula 1-3 (1-(2-cyclohexyl-1H-benzo[d]imidazol-5-yl)-3-(5-methoxy-2,2-dimethyl-2H-chromen-6-yl)urea)

The chromene-benzimidazole derivative of Formula 1-3 was synthesized as follows.

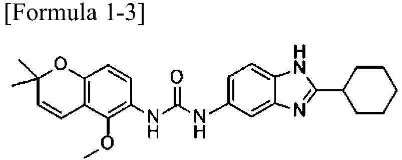

5-Methoxy-2,2-dimethyl-2H-chromene-6-carboxylic acid and 2-cyclohexyl-1H-benzo[d]imidazol-5-amine were reacted in a similar manner as in Method 1 of Example 1-1 to obtain Compound 1-3.

Yield 66%
¹H NMR (400 MHz, DMSO-*d*₆) δ 11.95(bs, 1H), 9.10(bs, 1H), 8.05(bd, 1H), 7.86(d, J=9.2Hz, 2H), 7.40(d, J=8.4Hz, 1H), 6.90(d, J=8.4Hz, 1H), 6.57(m, 2H), 5.83(d, J=9.6Hz, 1H), 3.73(s, 3H), 2.79(m, 1H), 2.02(m, 2H), 1.81(m, 2H), 1.68(m, 2H), 1.36(s, 6H), 1.27(m, 2H), 1.24(m, 2H)

### 1.4 Synthesis of Formula 1-4 (1-(5-methoxy-2,2-dimethyl-2H-chromen-6-yl)-3-(2-(4-methylpiperazin-1-yl)-1H-benzo[d]imidazol-5-yl)urea)

The chromene-benzimidazole derivative of Formula 1-4 was synthesized as follows.

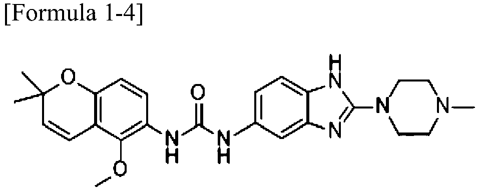

5-Methoxy-2,2-dimethyl-2H-chromene-6-carboxylic acid and 2-(4-methylpiperazin-1-yl)-1H-benzo[d]imidazol-5-amine were reacted in a similar manner as in Method 1 of Example 1-1 to obtain Compound 1-4.

Yield 48%
¹H NMR (400 MHz, DMSO-*d*₆) δ 7.78(s, 1H), 7.22(m, 1H), 7.12(m, 2H), 6.54(m, 2H), 5.78(d, J=10.0Hz, 1H), 3.65(s, 3H), 3.40(m, 4H0, 2.30(m, 4H), 2.19(s, 3H), 1.36(s, 6H)

### 1.5 Synthesis of Formula 1-5 (1-(1H-benzo[d]imidazol-5-yl)-3-(5-methoxy-2,2-dimethyl-2H-chromen-6-yl)urea)

The chromene-benzimidazole derivative of Formula 1-5 was synthesized as follows.

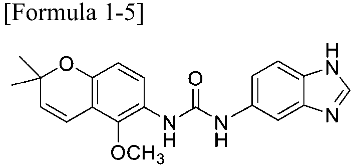

5-Methoxy-2,2-dimethyl-2H-chromene-6-carboxylic acid and 1H-benzo[d]imidazol-5-amine were reacted in a similar manner as in Method 1 of Example 1-1 to obtain Compound 1-5.

Yield 73%
¹H NMR (400 MHz, DMSO-*d*₆) δ 12.27(bs, 1H), 9.18(bs, 1H), 8.13(m, 2H), 7.85(d, J=8.8Hz, 1H), 7.53(d, J=8.4Hz, 1H), 7.24(m, 1H), 7.12(m, 1H), 6.55(m, 2H), 5.83(d, J=10.0Hz, 1H), 3.73(s, 3H), 1.37(s, 6H)

### 1.6 Synthesis of Formula 1-6 (1-(5-methoxy-2,2-dimethyl-2H-chromen-6-yl)-3-(2-(thiophen-2-yl)-1H-benzo[d]imidazol-5-yl)urea)

The chromene-benzimidazole derivative of Formula 1-6 was synthesized as follows.

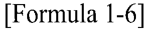

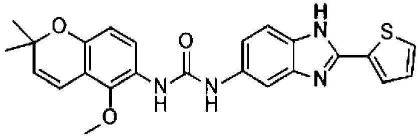

5-Methoxy-2,2-dimethyl-2H-chromene-6-carboxylic acid and 2-(thiophen-2-yl)-1H-benzo[d]imidazol-5-amine were reacted in a similar manner as in Method 1 of Example 1-1 to obtain Compound 1-6.

Yield 52%
¹H NMR (400 MHz, DMSO-*d*₆) δ 12.82(bs, 1H), 9.23(bs, 1H), 8.11(s, 1H), 7.95(s, 1H), 7.86-7.68(m, 3H), 7.51(d, J=8.8Hz, 1H), 7.39(m, 2H), 6.98(d, J=8.8Hz, 1H), 6.58(m, 2H), 5.84(d, J=10.0Hz,1H), 3.74(s, 3H), 1.37(s, 6H)

### 1.7 Synthesis of Formula 1-7 (1-(5-methoxy-2,2-dimethyl-2H-chromen-6-yl)-3-(2-(thiazol-4-yl)-1H-benzo[d]imidazol-5-yl)urea)

The chromene-benzimidazole derivative of Formula 1-7 was synthesized as follows.

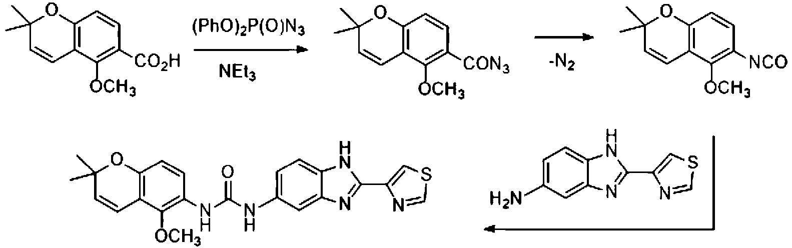

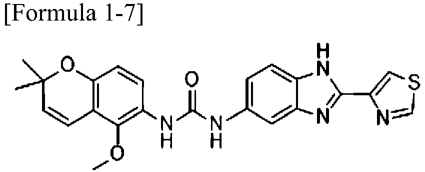

To a toluene (5 mL) solution of 5-Methoxy-2,2-dimethyl-2H-chromene-6-carboxylic acid (0.117 g, 0.5 mmole), triethylamine (0.068 g, 0.09 mL, 0.66 mmol) and diphenyl phosphoryl azide (DPPA, 0.210g, 0.763 mmoles, 1.5eq) were sequentially added, and the mixture was stirred at room temperature for 1 hour. Then, a previously prepared mixed solution of 2-(thiazol-4-yl)-1H-benzo[d]imidazol-5-amine (0.108 g, 0.51 mmol) and triethylamine (0.205 g, 0.276 mL, 2.02 mmol) was added, and the mixture was reacted at 110°C for 5 hours. The reaction mixture was cooled, the solvent was removed, and the product was purified by silica gel column chromatography to obtain the pure product compound 1-7 (0.08 g).

Yield 36%
¹H NMR (400 MHz, DMSO-*d*₆) δ 12.80(bs, 1H), 9.32(s, 1H), 9.22(bs, 1H), 8.41(s, 1H), 8.09(s, 1H), 7.94-7.84(m, 2H), 7.54(d, J=8.8Hz, 1H), 7.04(d, J=8.4Hz, 1H), 6.58(m, 2H), 5.84(d, J=10.0Hz, 1H), 3.74(s, 3H), 1.37(6H)

### 1.8 Synthesis of Formula 1-8 (1-(5-methoxy-2,2-dimethyl-2H-chromen-6-yl)-3-(2-phenyl-1H-benzo[d]imidazol-5-yl)urea)

The chromene-benzimidazole derivative of Formula 1-8 was synthesized as follows.

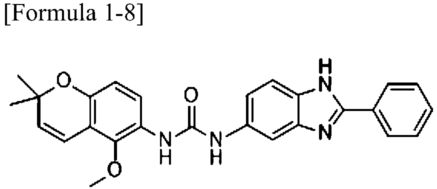

5-Methoxy-2,2-dimethyl-2H-chromene-6-carboxylic acid and 2-phenyl-1H-benzo[d]imidazol-5-amine were reacted in the same manner as in the method of Example 1-7, and the developing solvent was sequentially changed from ethyl acetate and hexane (1:1, v/v) to ethyl acetate and hexane (3:1, v/v) as purification solvents to obtain Compound 1-8.

Yield 72%
¹H NMR (400 MHz, DMSO-*d*₆) δ 12.79(bs, 1H), 9.23(bs, 1H), 8.11(m, 3H), 8.00(s, 1H), 7.87(d, J=8.8Hz, 1H), 5.56(m, 3H), 7.42(m, 1H), 6.98(m, 1H), 6.55(m, 2H), 5.84(d, J=10.0Hz, 1H), 3.74(s, 3H), 1.37(s, 6H)

### 1.9 Synthesis of Formula 1-9 (1-(2-(4-fluorophenyl)-1H-benzo[d]imidazol-5-yl)-3-(5-methoxy-2,2-dimethyl-2H-chromen-6-yl)urea)

The chromene-benzimidazole derivative of Formula 1-9 was synthesized as follows.

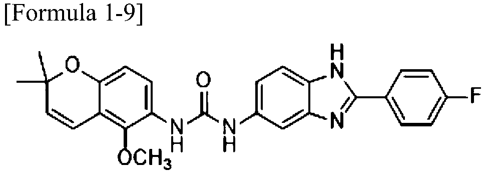

5-Methoxy-2,2-dimethyl-2H-chromene-6-carboxylic acid and 2-(4-fluorophenyl)-1H-benzo[d]imidazol-5-amine were reacted in the same manner as in the method of Example 1-7 to synthesize Compound 1-9.

Yield 50%
¹H NMR(400 MHz, DMSO-*d*₆) δ 12.80(s, 1H), 9.23(s, 1H), 8.16(m, 3H), 7.99(s, 1H), 7.84(d, J=8.8Hz, 1H), 7.54(d, J=8.8Hz, 1H), 7.39(m, 2H), 6.99(m, 1H), 6.55(m, 2H), 5.84(d, J=9.6Hz, 1H), 3.74(s, 3H), 1.37(s, 6H)

### 1.10 Synthesis of Formula 1-10 (1-(2-(3-fluorophenyl)-1H-benzo[d]imidazol-5-yl)-3-(5-methoxy-2,2-dimethyl-2H-chromen-6-yl)urea)

The chromene-benzimidazole derivative of Formula 1-10 was synthesized as follows.

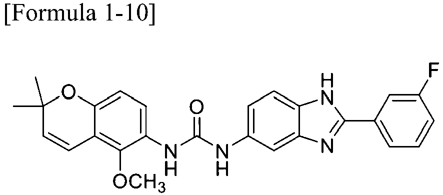

5-Methoxy-2,2-dimethyl-2H-chromene-6-carboxylic acid and 2-(3-fluorophenyl)-1H-benzo[d]imidazol-5-amine were reacted in the same manner as in the method of Example 1-7 to obtain Compound 1-10.

Yield: 67%
¹H NMR (400 MHz, DMSO-*d*₆) δ 11.82(s, 1H), 9.26(s, 1H), 8.12(s, 1H), 8.12~7.84(m, 4H), 7.58(m, 2H), 7.33(m, 1H), 6.98(d, J=8.8Hz, 1H), 6.58(t, 2H), 5.84(d, J=10.0Hz, 1H), 3.74(s, 3H), 1.37(s, 6H)

### 1.11 Synthesis of Formula 1-11 (1-(2-(4-chlorophenyl)-1H-benzo[d]imidazol-5-yl)-3-(5-methoxy-2,2-dimethyl-2H-chromen-6-yl)urea)

The chromene-benzimidazole derivative of Formula 1-11 was synthesized as follows.

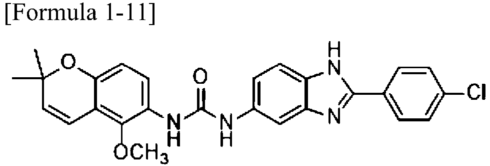

5-Methoxy-2,2-dimethyl-2H-chromene-6-carboxylic acid and 2-(4-chlorophenyl)-1H-benzo[d]imidazol-5-amine were reacted in the same manner as in the method of Example 1-7 to synthesize Compound 1-11.

Yield 33%
¹H NMR(400 MHz, DMSO-*d*₆) δ 12.80(s, 1H), 9.24(s, 1H), 8.14(m, 3H), 8.00(s, 1H), 7.86(d, J=7.2Hz, 1H), 7.63(d, J=7.2Hz, 2H), 7.57(m, 1H), 6.99(m, 1H), 6.58(t, 2H), 5.83(d, J=9.6Hz, 1H), 3.74(s, 3H), 1.37(s, 6H)

### 1.12 Synthesis of Formula 1-12 (1-(2-(4-hydroxyphenyl)-1H-benzo[d]imidazol-5-yl)-3-(5-methoxy-2,2-dimethyl-2H-chromen-6-yl)urea)

The chromene-benzimidazole derivative of Formula 1-12 was synthesized as follows.

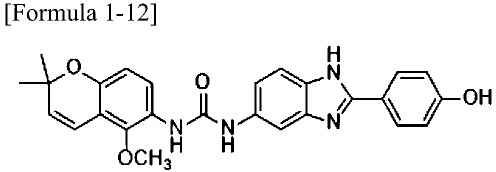

5-Methoxy-2,2-dimethyl-2H-chromene-6-carboxylic acid and 4-(5-amino-1H-benzo[d]imidazol-2-yl)phenol were reacted in the same manner as in the method of Example 1-7 to obtain Compound 1-12.

Yield 30%
¹H NMR(400 MHz, DMSO-*d*₆) δ 12.46(s, 1H), 9.91(bs, 1H), 9.18(s, 1H), 8.09(s, 1H), 7.95(m, 3H), 7.86(d, J=8.8Hz, 1H), 7.49(d, J=8.4Hz, 1H), 6.90(m, 3H), 6.55(m, 2H), 5.84(d, J=9.6Hz, 1H), 3.74(s, 3H), 1.37(s, 6H)

### 1.13 Synthesis of Formula 1-13 (1-(2-(4-cyanophenyl)-1H-benzo[d]imidazol-5-yl)-3-(5-methoxy-2,2-dimethyl-2H-chromen-6-yl)urea)

The chromene-benzimidazole derivative of Formula 1-13 was synthesized as follows.

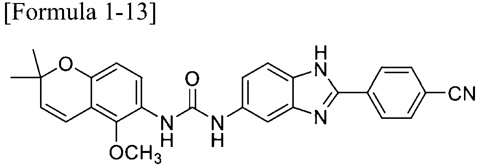

5-Methoxy-2,2-dimethyl-2H-chromene-6-carboxylic acid and 4-(5-amino-1H-benzo[d]imidazol-2-yl)benzonitrile were reacted in the same manner as in the method of Example 1-7 to obtain Compound 1-13.

Yield: 58%
¹H NMR (400 MHz, DMSO-*d*₆) δ 13.09(s, 1H), 9.29(s, 1H), 8.33(m, 2H), 8.13(s, 1H), 8.05(m, 3H), 7.86(d, J=8.8Hz, 1H), 7.62(d, J=9.2Hz, 1H), 7.02(d, J=9.2Hz, 1H), 6.58(t, 2H), 5.84(d, J=10.0Hz, 1H), 3.74(s, 3H), 1.37(s, 6H)

### 1.14 Synthesis of Formula 1-14 (1-(5-methoxy-2,2-dimethyl-2H-chromen-6-yl)-3-(2-(4-(trifluoromethyl)phenyl)-1H-benzo[d]imidazol-5-yl)urea)

The chromene-benzimidazole derivative of Formula 1-14 was synthesized as follows.

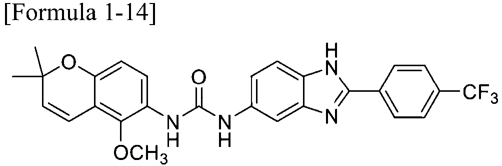

5-Methoxy-2,2-dimethyl-2H-chromene-6-carboxylic acid and 2-(4-(trifluoromethyl)phenyl)-1H-benzo[d]imidazol-5-amine were reacted in the same manner as in the method of Example 1-7 to obtain Compound 1-14 as a light brown solid.

Yield: 37%
¹H NMR (400 MHz, DMSO-*d*₆) δ 13.06(s, 1H), 9.28(s, 1H), 8.34(m, 2H), 8.13(s, 1H), 8.04(s, 1H), 7.93(d, J=8.8Hz, 2H), 7.87(d, J=8.8Hz, 1H), 7.61(d, J=8.8Hz, 1H), 7.03(d, J=10.4Hz, 1H), 6.58(t, 2H), 5.84(d, J=9.6Hz, 1H), 3.74(s, 3H), 1.37(s, 6H)

### 1.15 Synthesis of Formula 1-15 (1-(5-methoxy-2,2-dimethyl-2H-chromen-6-yl)-3-(2-(6-methoxypyridin-3-yl)-1H-benzo[d]imidazol-5-yl)urea)

The chromene-benzimidazole derivative of Formula 1-15 was synthesized as follows.

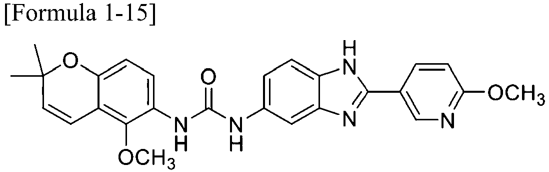

5-Methoxy-2,2-dimethyl-2H-chromene-6-carboxylic acid and 2-(6-methoxypyridin-3-yl)-1H-benzo[d]imidazol-5-amine were reacted in the same manner as in the method of Example 1-7 to obtain Compound 1-15 as a light brown solid.

Yield: 71%
¹H NMR (400 MHz, DMSO-*d*₆) δ 12.79(s, 1H), 9.23(s, 1H), 8.92(s, 1H), 8.37(m, 1H), 8.08(s, 1H), 8.00(s, 1H), 7.86(d, J=8.8Hz, 1H), 7.55(d, J=8.4Hz, 1H), 7.01(d, J=8.8Hz, 2H), 6.55(t, 2H), 5.84(d, J=9.6Hz, 1H), 3.94(s, 3H), 3.74(s, 3H), 1.37(s, 6H)

### 1.16 Synthesis of Formula 1-16 (1-(2-(4-(dimethylamino)phenyl)-1H-benzo[d]imidazol-5-yl)-3-(5-methoxy-2,2-dimethyl-2H-chromen-6-yl)urea)

The chromene-benzimidazole derivative of Formula 1-16 was synthesized as follows.

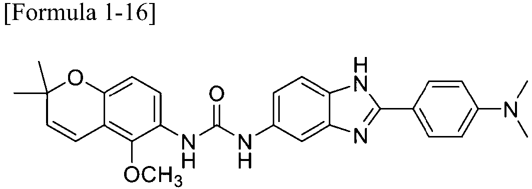

5-Methoxy-2,2-dimethyl-2H-chromene-6-carboxylic acid and 2-(4-(dimethylamino)phenyl)-1H-benzo[d]imidazol-5-amine were reacted in the same manner as in the method of Example 1-7 to obtain Compound 1-16 as a light brown solid.

Yield: 36%
¹H NMR (400 MHz, DMSO-*d*₆) δ 12.36(s, 1H), 9.16(s, 1H), 8.08(s, 1H), 7.97(m, 3H), 7.87(d, J=8.4Hz, 1H), 7.46(d, J=8.0Hz, 1H), 6.93(d, J=7.6Hz, 1H), 6.84(d, J=8.4Hz, 2H), 6.589t, 2H), 5.84(d, J=9.6Hz, 1H), 3.74(s, 3H), 2.99(s, 6H), 1.37(s, 6H)

### 1.17 Synthesis of Formula 1-17 (1-(5-methoxy-2,2-dimethyl-2H-chromen-6-yl)-3-(2-(3,4,5-trimethoxyphenyl)-1H-benzo[d]imidazol-5-yl)urea)

The chromene-benzimidazole derivative of Formula 1-17 was synthesized as follows.

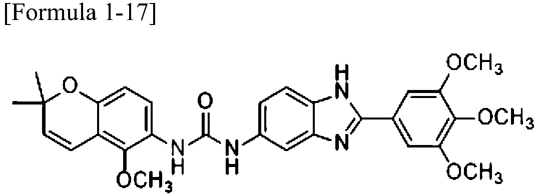

5-Methoxy-2,2-dimethyl-2H-chromene-6-carboxylic acid and 2-(3,4,5-trimethoxyphenyl)-1H-benzo[d]imidazol-5-amine were reacted in the same manner as in the method of Example 1-7 to synthesize Compound 1-17.

Yield 23%
¹H NMR(400 MHz, DMSO-*d*₆) δ 12.71(s, 1H), 9.23(ss, 1H), 8.11(s, 1H), 8.00(s, 1H), 7.86(m, 1H), 7.49(m, 3H), 6.98(d, J=8.0Hz, 1H), 6.58(m, 2H), 5.84(d, J=9.6Hz, 1H), 3.90(s, 6H), 3.74(d, J=4.8Hz, 6H), 1.37(s, 6H)

### 1.18 Synthesis of Formula 1-18 (1-(2-(3,5-dimethoxyphenyl)-1H-benzo[d]imidazol-5-yl)-3-(5-methoxy-2,2-dimethyl-2H-chromen-6-yl)urea)

The chromene-benzimidazole derivative of Formula 1-18 was synthesized as follows.

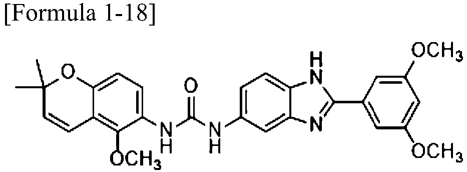

5-Methoxy-2,2-dimethyl-2H-chromene-6-carboxylic acid and 2-(3,5-dimethoxyphenyl)-1H-benzimidazol-5-amine were reacted in the same manner as in the method of Example 1-7 to obtain Compound 1-18.

Yield 57%
¹H NMR (400 MHz, DMSO-*d*₆) δ 12.71(bs, 1H), 9.24(bs, 1H), 8.11(bs, 1H), 8.00(s, 1H), 7.85(m, 1H), 7.57(d, J=8.4Hz, 1H), 7.32(s, 2H), 6.969d, J=8.8Hz, 1H), 6.55(m, 3H), 5.84(d, J=8.8Hz, 1H), 3.85(s, 6H), 3.74(s, 3H), 1.37(s, 6H)

### 1.19 Synthesis of Formula 1-19 (1-(2-(2,4-difluorophenyl)-1H-benzo[d]imidazol-5-yl)-3-(5-methoxy-2,2-dimethyl-2H-chromen-6-yl)urea)

The chromene-benzimidazole derivative of Formula 1-19 was synthesized as follows.

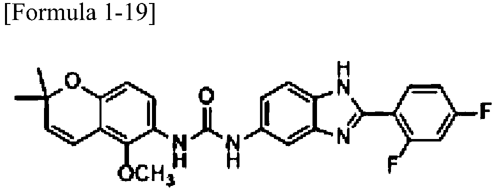

5-Methoxy-2,2-dimethyl-2H-chromene-6-carboxylic acid and 2-(2,4-difluorophenyl)-1H-benzo[d]imidazol-5-amine were reacted in the same manner as in the method of Example 1-7 to obtain Compound 1-19.

Yield 50%
¹H NMR (400 MHz, DMSO-*d*₆) δ 12.43(s, 1H), 9.25(s, 1H), 8.26(m, 1H), 8.22(s, 1H), 8.09(s, 1H), 7.87(d, J=8.8Hz, 1H), 7.51(m, 2H), 7.32(m, 1H), 7.05(d, J=8.8Hz, 1H), 6.55(m, 2H), 5.84(d, J=10.0Hz, 1H), 3.74(s, 3H), 1.37(s, 3H)

### 1.20 Synthesis of Formula 1-20 (1-(2-(2,4-dichlorophenyl)-1H-benzo[d]imidazol-5-yl)-3-(5-methoxy-2,2-dimethyl-2H-chromen-6-yl)urea)

The chromene-benzimidazole derivative of Formula 1-20 was synthesized as follows.

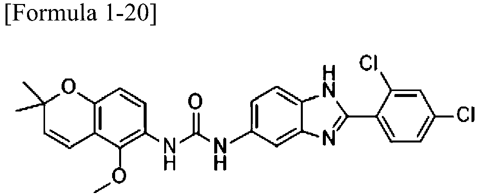

5-Methoxy-2,2-dimethyl-2H-chromene-6-carboxylic acid and 2-(2,4-dichlorophenyl)-1H-benzo[d]imidazol-5-amine were reacted in the same manner as in the method of Example 1-7 to obtain Compound 1-20.

Yield 53%
¹H NMR (400 MHz, DMSO-*d*₆) δ 12.59(bs, 1H), 9.26(bs, 1H), 8.12(bs, 1H), 8.049s, 1H), 7.95(q, 1H), 7.86(s, 2H), 7.58(m, 2H), 7.00(d, J=8.4Hz, 1H), 6.54(m, 2H), 5.84(d, J=10.0Hz, 1H),
3.74(s, 3H), 1.37(s, 6H)

### 1.21 Synthesis of Formula 1-21 (1-(2-(2,3-dichlorophenyl)-1H-benzo[d]imidazol-5-yl)-3-(5-methoxy-2,2-dimethyl-2H-chromen-6-yl)urea)

The chromene-benzimidazole derivative of Formula 1-21 was synthesized as follows.

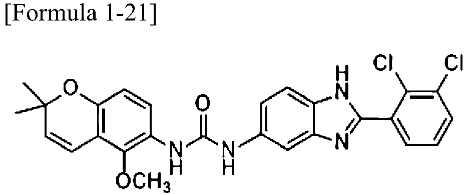

5-Methoxy-2,2-dimethyl-2H-chromene-6-carboxylic acid and 2-(2,3-dichlorophenyl)-1H-benzo[d]imidazol-5-amine were reacted in the same manner as in the method of Example 1-7 to obtain Compound 1-21.

Yield 59%
¹H NMR (400 MHz, DMSO-*d*₆) δ 12.66(bs, 1H), 9.30(bs, 1H), 8.15(s, 1H), 8.04(s, 1H), 7.84(m, 3H), 7.59(m, 2H), 7.03(m, 1H), 6.55(m, 2H), 5.84(d, J=9.6Hz, 1H), 3.74(s, 3H), 1.37(s, 6H)

### 1.22 Synthesis of Formula 1-22 (1-(5-methoxy-2,2-dimethyl-2H-chromen-6-yl)-3-(2-(p-tolyl)-1H-benzo[d]imidazol-5-yl)urea)

The chromene-benzimidazole derivative of Formula 1-22 was synthesized as follows.

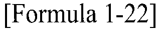

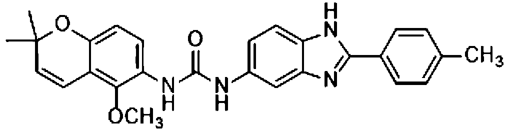

5-Methoxy-2,2-dimethyl-2H-chromene-6-carboxylic acid and 2-(p-tolyl)-1H-benzo[d]imidazol-5-amine were reacted in the same manner as in the method of Example 1-7 to obtain Compound 1-22.

Yield 81%
¹H NMR(400 MHz, DMSO-*d*₆) δ 12.69(s, 1H), 9.22(s, 1H), 8.02(m, 4H), 7.84(m,1H), 7.54(d, J=8.4Hz, 1H), 7.359d, J=7.6Hz, 2H), 6.98(d, J=8.8Hz, 1H), 6.55(m, 2H), 5.84(d, J=10.0Hz, 1H), 3.74(s, 3H), 2.38(s, 3H), 1.37(s, 6H)

### 1.23 Synthesis of Formula 1-23 (1-(2-(4-isopropylphenyl)-1H-benzo[d]imidazol-5-yl)-3-(5-methoxy-2,2-dimethyl-2H-chromen-6-yl)urea)

The chromene-benzimidazole derivative of Formula 1-23 was synthesized as follows.

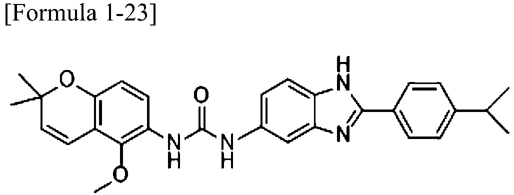

5-Methoxy-2,2-dimethyl-2H-chromene-6-carboxylic acid and 2-(4-isopropylphenyl)-1H-benzo[d]imidazol-5-amine were reacted in the same manner as in the method of Example 1-7 to obtain Compound 1-23.

Yield 74%
¹H NMR (400 MHz, DMSO-*d*₆) δ 12.70(bs, 1H), 9.23(bs, 1H), 8.12(m, 3H), 7.98(s, 1H), 7.86(d, J=8.8Hz, 1H), 7.54(d, J=8.4Hz, 1H), 7.42(d, J=8.4Hz, 2H), 6.98(d, J=8.8Hz, 1H), 6.55(m, 2H), 5.84(d, J=10.0Hz, 1H), 3.74(s, 3H), 2.96(q, 1H), 1.379s, 6H), 1.26(d, J=6.8Hz, 6H)

### 1.24 Synthesis of Formula 1-24 (1-(2-(4-(tert-butyl)phenyl)-1H-benzo[d]imidazol-5-yl)-3-(5-methoxy-2,2-dimethyl-2H-chromen-6-yl)urea)

The chromene-benzimidazole derivative of Formula 1-24 was synthesized as follows.

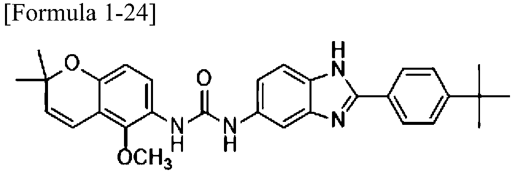

5-Methoxy-2,2-dimethyl-2H-chromene-6-carboxylic acid and 2-(4-tert-butylphenyl)-1H-benzimidazol-5-amine were reacted in the same manner as in the method of Example 1-7 to obtain Compound 1-24.

Yield 55%
¹H NMR (400 MHz, DMSO-*d*₆) δ 12.65(bs, 1H), 9.21(bs, 1H), 8.10(m, 3H), 7.98(s, 1H), 7.85(m, 1H), 7.57(m, 3H), 6.98(d, J=8.4Hz, 1H), 6.58(t, 2H), 5.84(d, J=10.0Hz, 1H), 3.74(s, 3H), 1.37(s, 6H), 1.33(s, 9H)

### 1.25 Synthesis of Formula 1-25 (1-(5-methoxy-2,2-dimethyl-2H-chromen-6-yl)-3-(2-(4-phenoxyphenyl)-1H-benzo[d]imidazol-5-yl)urea)

The chromene-benzimidazole derivative of Formula 1-25 was synthesized as follows.

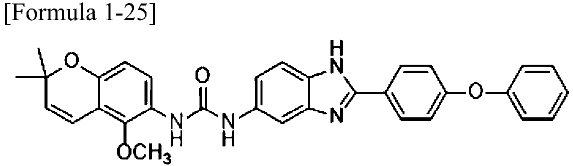

5-Methoxy-2,2-dimethyl-2H-chromene-6-carboxylic acid and 2-(4-phenoxyphenyl)-1H-1,3-benzodiazol-5-amine were reacted in the same manner as in the method of Example 1-7 to obtain Compound 1-25.

Yield 63%
¹H NMR (400 MHz, DMSO-*d*₆) δ 12.73(bs, 1H), 9.22(bs, 1H), 8.15(m, 3H), 7.84(m, 1H), 7.45(m, 3H), 7.25(m, 2H), 7.18(m, 3H), 6.55(m, 2H), 5.84(d, J=10.0Hz, 1H), 3.74(s, 3H), 1.37(s, 6H)

### 1.26 Synthesis of Formula 1-26 (1-(2-(3-chloro-4-fluorophenyl)-1H-benzo[d]imidazol-5-yl)-3-(5-methoxy-2,2-dimethyl-2H-chromen-6-yl)urea)

The chromene-benzimidazole derivative of Formula 1-26 was synthesized as follows.

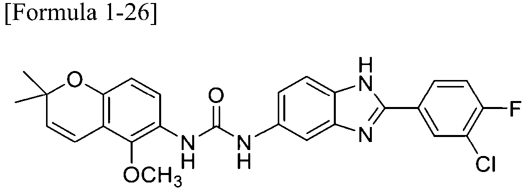

5-Methoxy-2,2-dimethyl-2H-chromene-6-carboxylic acid and 2-(3-chloro-4-fluorophenyl)-1H-benzo[d]imidazol-5-amine were reacted in the same manner as in the method of Example 1-7 to obtain Compound 1-26.

Yield 70%
¹H NMR (400 MHz, DMSO-*d*₆) δ 12.84(bs, 1H), 9.26(bs, 1H), 8.31(s, 1H), 8.12(s, 2H), 8.00(bs, 1H), 7.86(d, J=8.8Hz, 1H), 7,74(m, 1H), 7.63(m, 2H), 7.01(bs, 1H), 6.55(m, 2H), 5.84(d, J=10.0Hz, 1H), 3.74(s, 3H), 1.37(s, 6H)

### 1.27 Synthesis of Formula 1-27 (1-(2-(4-(benzyloxy)phenyl)-1H-benzo[d]imidazol-5-yl)-3-(5-methoxy-2,2-dimethyl-2H-chromen-6-yl)urea)

The chromene-benzimidazole derivative of Formula 1-27 was synthesized as follows.

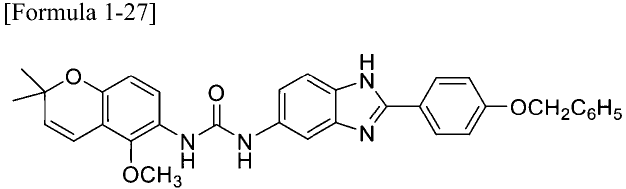

5-Methoxy-2,2-dimethyl-2H-chromene-6-carboxylic acid and 2-(4-(benzyloxy)phenyl)-1H-benzo[d]imidazol-5-amine were reacted in the same manner as in the method of Example 1-7 to obtain Compound 1-27.

Yield 53%
¹H NMR (400 MHz, DMSO-*d*₆) δ 12.62(bs, 1H), 9.21(bs, 1H), 8.10(m, 3H), 7.96(s, 1H), 7.86(d, J=8.8Hz, 1H), 7.53(m, 3H), 7.35(m, 3H), 7.19(d, J=8.4Hz, 2H), 6.96(d, J=8.4Hz, 1H), 6.58(m, 2H), 5.84(d, J=9.6Hz, 1H), 5.20(s, 2H), 3.74(s, 3H), 1.37(s, 6H)

### 1.28 Synthesis of Formula 1-28 (1-(2-(4-(diethylamino)phenyl)-1H-benzo[d]imidazol-5-yl)-3-(5-methoxy-2,2-dimethyl-2H-chromen-6-yl)urea)

The chromene-benzimidazole derivative of Formula 1-28 was synthesized as follows.

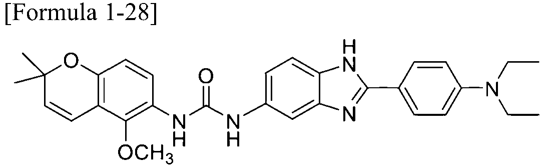

5-Methoxy-2,2-dimethyl-2H-chromene-6-carboxylic acid and 2-(4-(diethylamino)phenyl)-1H-benzo[d]imidazol-5-amine were reacted in the same manner as in the method of Example 1-7 to obtain Compound 1-28.

Yield 54%
¹H NMR (400 MHz, DMSO-*d*₆) δ 12.35(bs, 1H), 9.15(bs, 1H), 8.08(bs, 1H), 7.89(m, 4H), 7.44(d, J=8.0Hz, 1H), 6.92(d, J=9.2Hz, 1H), 6.789d, J=8.4Hz, 2H), 6.55(m, 2H), 5.84(d, J=10.0Hz, 1H), 3.74(s, 3H), 3.42(q, 4H), 1.37(s, 6H), 1.15(t, 6H)

### 1.29 Synthesis of Formula 1-29 (1-(2-(3,4-difluorophenyl)-1H-benzo[d]imidazol-5-yl)-3-(5-methoxy-2,2-dimethyl-2H-chromen-6-yl)urea)

The chromene-benzimidazole derivative of Formula 1-29 was synthesized as follows.

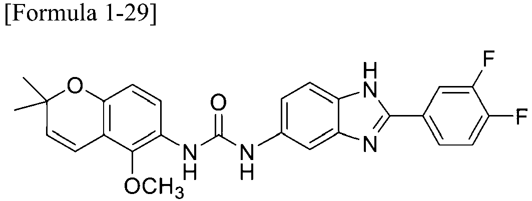

5-Methoxy-2,2-dimethyl-2H-chromene-6-carboxylic acid and 2-(3,4-difluorophenyl)-1H-benzo[d]imidazol-5-amine were reacted in the same manner as in the method of Example 1-7 to obtain Compound 1-29.

Yield 57%
¹H NMR (400 MHz, DMSO-*d*₆) δ 12,84(bs, 1H), 9.26(bs, 1H), 8.12(s, 2H), 8.00(s, 2H), 7.86(d, J=8.8Hz, 1H), 7.65(m, 1H), 7.55(m, 1H), 7.00(m, 1H), 6.55(m, 2H), 5.84(d, J=10.0Hz, 1H), 3.74(s, 3H), 1.37(s, 6H)

### 1.30 Synthesis of Formula 1-30 (1-(5-methoxy-2,2-dimethyl-2H-chromen-6-yl)-3-(2-(4-(pentyloxy)phenyl)-1H-benzo[d]imidazol-5-yl)urea)

The chromene-benzimidazole derivative of Formula 1-30 was synthesized as follows.

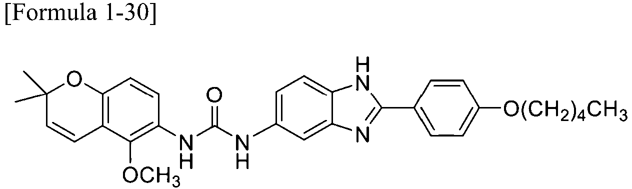

5-Methoxy-2,2-dimethyl-2H-chromene-6-carboxylic acid and 2-(4-(pentyloxy)phenyl)-1H-benzo[d]imidazol-5-amine were reacted in the same manner as in the method of Example 1-7 to obtain Compound 1-30.

Yield 60%
¹H NMR (400 MHz, DMSO-*d*₆) δ 12.60(bs, 1H), 9.19(bs, 1H), 8.09(m, 3H), 7.95(s, 1H), 7.87(d, J=8.8Hz, 1H), 7.51(d, J=8.4Hz, 1H), 7.09(d, J=8.8Hz, 2H), 6.96(d, J=8.4Hz, 1H), 6.55(m, 2H), 5.84(d, J=10.0Hz, 1H), 4.06(m, 2H), 3.74(s, 3H), 1.77(m, 2H), 1.35(m, 4H), 1.37(s, 6H), 0.93(t, 3H)

### 1.31 Synthesis of Formula 1-31 (1-(2-(5-chloro-2-hydroxyphenyl)-1H-benzo[d]imidazol-5-yl)-3-(5-methoxy-2,2-dimethyl-2H-chromen-6-yl)urea)

The chromene-benzimidazole derivative of Formula 1-31 was synthesized as follows.

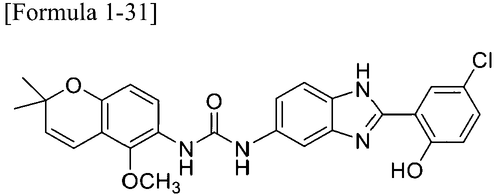

5-Methoxy-2,2-dimethyl-2H-chromene-6-carboxylic acid and 2-(5-amino-1H-benzo[d]imidazol-2-yl)-4-chlorophenol were reacted in the same manner as in the method of Example 1-7 to obtain Compound 1-31.

Yield 59%
¹H NMR (400 MHz, DMSO-*d*₆) δ 13.26(bs, 1H), 9.28(bs, 1H), 8.13(d, J=12.0Hz, 2H), 8.02(s, 1H), 7.86(d, J=8.8Hz, 1H), 7.60(bs, 1H), 7.39(d, J=8.8Hz, 1H), 7.12(m, 1H), 7.06(d, J=8.8Hz, 2H), 6.58(m, 2H), 5.84(d, J=10.0Hz, 1H), 3.74(s, 3H), 1.37(s, 6H)

### 1.32 Synthesis of Formula 1-32 (1-(2-(4-(1H-indol-3-yl)phenyl)-1H-benzold]imidazol-5-yl)-3-(5-methoxy-2,2-dimethyl-2H-chromen-6-yl)urea)

The chromene-benzimidazole derivative of Formula 1-32 was synthesized as follows.

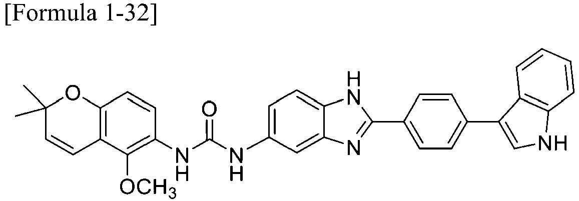

5-Methoxy-2,2-dimethyl-2H-chromene-6-carboxylic acid and 2-(4-(1H-indol-3-yl)phenyl)-1H-benzo[d]imidazol-5-amine were reacted in the same manner as in the method of Example 1-7 to obtain Compound 1-32.

Yield 38%
¹H NMR (400 MHz, DMSO-*d*₆) δ 12.28(bs, 1H), 11.58(m, 1H), 9.16(bs, 1H), 8.48(m, 1H), 8.10(m, 2H), 7.91(m, 2H), 7.21(m, 3H), 6.95(d, J=8.4Hz, 1H), 6.58(m, 2H), 5.84(d, J=9.6Hz, 1H), 3.74(s, 3H), 1.37(s, 6H)

### 1.33 Synthesis of Formula 1-33 (1-(5-methoxy-2,2-dimethyl-2H-chromen-6-yl)-3-(2-(4-propylphenyl)-1H-benzo[d]imidazol-5-yl)urea)

The chromene-benzimidazole derivative of Formula 1-33 was synthesized as follows.

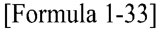

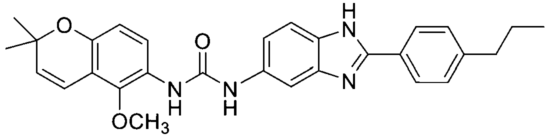

5-Methoxy-2,2-dimethyl-2H-chromene-6-carboxylic acid and 2-(4-propylphenyl)-1H-benzo[d]imidazol-5-amine were reacted in the same manner as in the method of Example 1-7 to obtain Compound 1-33.

Yield 72%
¹H NMR (400 MHz, DMSO-*d*₆) δ 12.70(bs, 1H), 9.21(bs, 1H), 8.10(m, 4H), 7.86(m, 1H), 7.54(d, J=8.8Hz, 1H), 7.36(m, 3H), 6.97(d, J=8.4Hz, 1H), 6.55(t, 2H), 5.84(d, J=10.4Hz, 1H), 3.74(s, 3H), 2.62(m, 2H), 1.65(m, 2H), 1.37(s, 6H), 0.94(t, 3H)

### 1.34 Synthesis of Formula 1-34 (1-(5-methoxy-2,2-dimethyl-2H-chromen-6-yl)-3-(2-(4-(4-methylpiperazin-1-yl)phenyl)-1H-benzo[d]imidazol-5-yl)urea)

The chromene-benzimidazole derivative of Formula 1-34 was synthesized as follows.

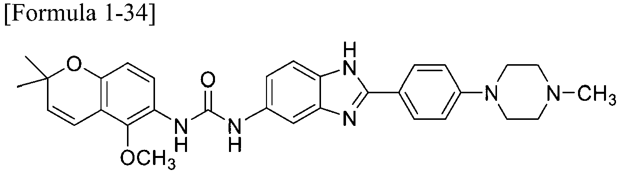

5-Methoxy-2,2-dimethyl-2H-chromene-6-carboxylic acid and 2-(4-(4-methylpiperazin-1-yl)phenyl)-1H-benzo[d]imidazol-5-amine were reacted in the same manner as in the method of Example 1-7 to obtain Compound 1-34.

Yield 58%
¹H NMR (400 MHz, DMSO-*d*₆) δ 12.59(bs, 1H), 9.24(bs, 1H), 8.11(s, 1H), 8.04(d, J=8.8Hz, 2H), 7.91(m, 1H), 7.86(d, J=8.8Hz, 2H), 7.46(bs, 1H), 7.16(m, 3H), 6.55(m, 2H), 5.84(d, J=10.0Hz, 1H), 4.02(m, 4H), 3.74(s, 3H), 3.10(m, 4H), 2.79(s, 3H), 1.37(s, 6H)

### 1.35 Synthesis of Formula 1-35 (1-(5-methoxy-2,2-dimethyl-2H-chromen-6-yl)-3-(2-(o-tolyl)-1H-benzo[d]imidazol-5-yl)urea)

The chromene-benzimidazole derivative of Formula 1-35 was synthesized as follows.

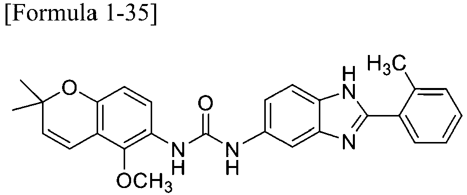

5-Methoxy-2,2-dimethyl-2H-chromene-6-carboxylic acid and 2-(o-tolyl)-1H-benzo[d]imidazol-5-amine were reacted in the same manner as in the method of Example 1-7 to obtain Compound 1-35.

Yield 73%
¹H NMR (400 MHz, DMSO-*d*₆) δ 12.44(bs, 1H), 9.23(bs, 1H), 8.12(s, 1H), 8.00(s, 1H), 7.87(m, 1H), 7.74(d, J=7.4Hz, 1H), 7.57(d, J=8.0Hz, 1H), 7.37(s, 3H), 6.99(d, J=8.8Hz, 1H), 6.58(m, 2H), 5.84(d, J=9.6Hz, 1H), 3.74(s, 3H), 2.61(s, 3H), 1.37(s, 6H)

### 1.36 Synthesis of Formula 1-36 (1-(5-methoxy-2,2-dimethyl-2H-chromen-6-yl)-3-(2-(m-tolyl)-1H-benzo[d]imidazol-5-yl)urea)

The chromene-benzimidazole derivative of Formula 1-36 was synthesized as follows.

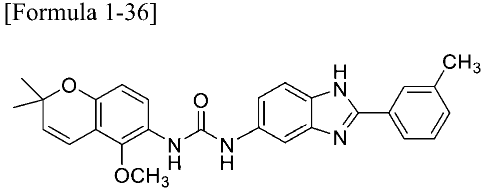

5-Methoxy-2,2-dimethyl-2H-chromene-6-carboxylic acid and 2-(m-tolyl)-1H-benzo[d]imidazol-5-amine were reacted in the same manner as in the method of Example 1-7 to obtain Compound 1-36.

Yield 68%
¹H NMR (400 MHz, DMSO-*d*₆) δ 12.75(bs, 1H), 9.23(bs, 1H), 8.11(s, 1H), 7.98(s, 2H), 7.92(m, 1H), 7.87(m, 1H), 7.55(d, J=8.4Hz, 1H), 7.42(m, 1H), 7.29(m, 1H), 6.99(d, J=8.0Hz, 1H), 6.55(m, 2H), 5.84(d, J=10.0Hz, 1H), 3.74(s, 3H), 2.41(s, 3H), 1.37(s, 6H)

### 1.37 Synthesis of Formula 1-37 (1-(5-methoxy-2,2-dimethyl-2H-chromen-6-yl)-3-(2-(3-methoxyphenyl)-1H-benzo[d]imidazol-5-yl)urea)

The chromene-benzimidazole derivative of Formula 1-37 was synthesized as follows.

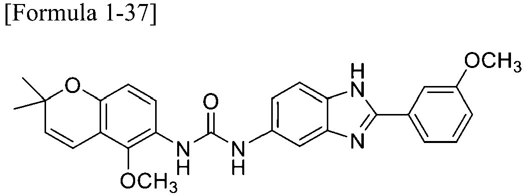

5-Methoxy-2,2-dimethyl-2H-chromene-6-carboxylic acid and 2-(3-methoxyphenyl)-1H-benzo[d]imidazol-5-amine were reacted in the same manner as in the method of Example 1-7 to obtain Compound 1-37.

Yield 67%
¹H NMR (400 MHz, DMSO-*d*₆) δ 12.01(s, 1H), 9.21(bs, 1H), 8.30(m, 1H), 8.12(m, 2H), 7.89(m, 1H), 7.54(d, J=8.4Hz, 1H), 7.47(m, 1H), 7.23(m, 1H), 7.11(m, 1H), 6.98(d, J=8.4Hz, 1H) 6.58(m, 2H), 5.84(d, J=10.0Hz, 1H), 4.02(s, 3H), 3.74(s, 3H), 1.37(s, 6H)

### 1.38 Synthesis of Formula 1-38 (1-(5-methoxy-2,2-dimethyl-2H-chromen-6-yl)-3-(2-(3-(trifluoromethyl)phenyl)-1H-benzo[d]imidazol-5-yl)urea)

The chromene-benzimidazole derivative of Formula 1-38 was synthesized as follows.

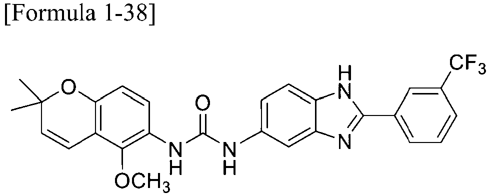

5-Methoxy-2,2-dimethyl-2H-chromene-6-carboxylic acid and 2-(3-(trifluoromethyl)phenyl)-1H-benzo[d]imidazol-5-amine were reacted in the same manner as in the method of Example 1-7 to obtain Compound 1-38.

Yield 61%
¹H NMR (400 MHz, DMSO-*d*₆) δ 12.99(bs, 1H), 9.28(bs, 1H), 8.47(m, 2H), 8.13(s, 1H), 8.03(s, 1H), 7.83(m, 3H), 7.60(d, J=8.0Hz, 1H), 7.03(d, J=8.0Hz, 1H), 6.58(t, 2H), 5.84(d, J=10.0Hz, 1H), 3.74(s, 3H), 1.37(s, 6H)

### 1.39 Synthesis of Formula 1-39 (1-(2-(4-benzoylphenyl)-1H-benzo[d]imidazol-5-yl)-3-(5-methoxy-2,2-dimethyl-2H-chromen-6-yl)urea)

The chromene-benzimidazole derivative of Formula 1-39 was synthesized as follows.

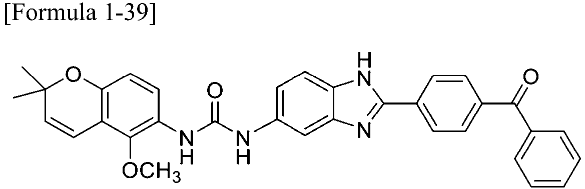

5-Methoxy-2,2-dimethyl-2H-chromene-6-carboxylic acid and (4-(5-amino-1H-benzo[d]imidazol-2-yl)phenyl)(phenyl)methanone were reacted in the same manner as in the method of Example 1-7 to obtain Compound 1-39.

Yield 73%
¹H NMR (400 MHz, DMSO-*d*₆) δ 12.99(bs, 1H), 9.28(bs, 1H), 8.32(d, J=7.6Hz, 2H), 8.13(s, 1H), 8.04(bs, 1H), 7.92(d, J=8.0Hz, 2H), 7.87(, J=8.8Hz, 1H), 7.81(d, J=8.0hz, 2H), 7.72(m, 1H), 7.61(m, 2H), 7.02(bs, 1H), 6.55(m, 2H), 5.84(d, J=9.6Hz, 1H), 3.74(s, 3H), 1.37(s, 6H)

### 1.40 Synthesis of Formula 1-40 (1-(2-(3,5-bis(trifluoromethyl)phenyl)-1H-benzo[d]imidazol-5-yl)-3-(5-methoxy-2,2-dimethyl-2H-chromen-6-yl)urea)

The chromene-benzimidazole derivative of Formula 1-40 was synthesized as follows.

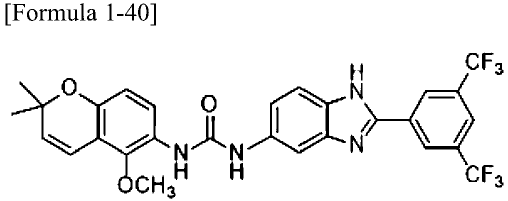

5-Methoxy-2,2-dimethyl-2H-chromene-6-carboxylic acid and 2-(3,5-bis(trifluoromethyl)phenyl)-1H-benzo[d]imidazol-5-amine were reacted in the same manner as in the method of Example 1-7 to obtain Compound 1-40.

Yield 75%
¹H NMR (400 MHz, DMSO-*d*₆) δ 13.21(bs, 1H), 9.31(bs, 1H), 8.76(s, 2H), 8.23(s, 1H), 8.14(s, 1H), 8.05(bs, 1H), 7.87(d, J=8.8Hz, 1H), 7.64(bs. 1H), 7.04(bs, 1H), 6.56(m, 2H), 5.84(d, J=10.0Hz, 1H), 3.74(s, 3H), 1.37(s, 6H)

### 1.41 Synthesis of Formula 1-41 (1-(5-methoxy-2,2-dimethyl-2H-chromen-6-yl)-3-(2-(4-(trifluoromethoxy)phenyl)-1H-benzo[d]imidazol-5-yl)urea)

The chromene-benzimidazole derivative of Formula 1-41 was synthesized as follows.

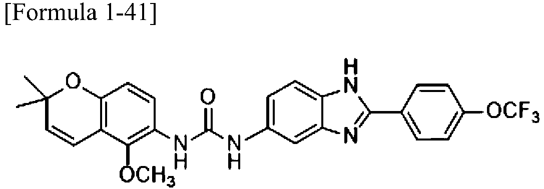

5-Methoxy-2,2-dimethyl-2H-chromene-6-carboxylic acid and 22-(4-(trifluoromethoxy)phenyl)-1H-benzo[d]imidazol-5-amine were reacted in the same manner as in the method of Example 1-7 to obtain Compound 1-41.

Yield 69%
¹H NMR (400 MHz, DMSO-*d*₆) δ 12.85(bs, 1H), 9.26(bs, 1H), 8.25(m, 3H), 8.12(bs, 1H), 8.02(s, 1H), 7.87(d, J=9.2Hz, 1H), 7.56(m, 3H), 7.00(d, J=8.4Hz, 1H), 6.55(m, 2H), 5.84(d, J=10.0Hz, 1H), 3.74(s, 3H), 1.37(s, 6H)

### 1.42 Synthesis of Formula 1-42 (1-(5-methoxy-2,2-dimethyl-2H-chromen-6-yl)-3-(2-(3-(trifluoromethoxy)phenyl)-1H-benzo[d]imidazol-5-yl)urea)

The chromene-benzimidazole derivative of Formula 1-42 was synthesized as follows.

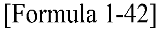

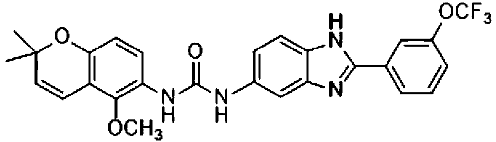

5-Methoxy-2,2-dimethyl-2H-chromene-6-carboxylic acid and 2-(3-(trifluoromethoxy)phenyl)-1H-benzo[d]imidazol-5-amine were reacted in the same manner as in the method of Example 1-7 to obtain Compound 1-42.

Yield 66%
¹H NMR (400 MHz, DMSO-*d*₆) δ 12.92(bs, 1H), 9.27(bs, 1H), 8.14(m, 3H), 8.08(s, 1H), 7.86(d, J=8.8Hz, 1H), 7.71(m, 1H), 7.67(m, 1H), 7.58(d, J=8.4Hz, 1H), 7.02(d, J=9.6Hz, 1H), 6.55(m, 2H), 5.84(d, J=10.0Hz, 1H), 3.74(s, 3H), 1.37(s, 6H)

### 1.43 Synthesis of Formula 1-43 (1-(2-(4-cyclopropylphenyl)-1H-benzo[d]imidazol-5-yl)-3-(5-methoxy-2,2-dimethyl-2H-chromen-6-yl)urea)

The chromene-benzimidazole derivative of Formula 1-43 was synthesized as follows.

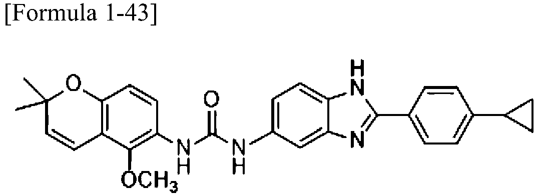

5-Methoxy-2,2-dimethyl-2H-chromene-6-carboxylic acid and 2-(4-cyclopropylphenyl)-1H-benzo[d]imidazol-5-amine were reacted in the same manner as in the method of Example 1-7 to obtain Compound 1-43.

Yield 69%
¹H NMR (400 MHz, DMSO-*d*₆) δ 12.68(bs, 1H), 9.21(bs, 1H), 8.10(bs, 1H), 7.98(m, 3H), 7.87(d, J=8.8Hz, 1H), 7.53(d, J=8.4Hz, 1H), 7.24(d, J=8.4Hz, 2H), 6.97(d, J=8.4Hz, 1H), 6.55(m, 2H), 5.84(d, J=10.0Hz, 1H), 3.74(s, 3H), 1.99(m, 1H), 1.37(s, 6H), 1.02(m, 2H), 0.77(m, 2H)

### 1.44 Synthesis of Formula 1-44 (1-(2-(4-cyclopentylphenyl)-1H-benzo[d]imidazol-5-yl)-3-(5-methoxy-2,2-dimethyl-2H-chromen-6-yl)urea)

The chromene-benzimidazole derivative of Formula 1-44 was synthesized as follows.

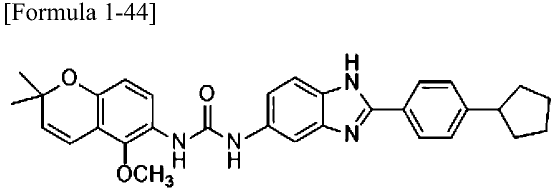

5-Methoxy-2,2-dimethyl-2H-chromene-6-carboxylic acid and 2-(4-cyclopentylphenyl)-1H-benzo[d]imidazol-5-amine were reacted in the same manner as in the method of Example 1-7 to obtain Compound 1-44.

Yield 71%
¹H NMR (400 MHz, DMSO-*d*₆) δ 12.70(bs, 1H), 9.22(bs, 1H), 8.10(m, 4H), 7.86(d, J=8.8Hz, 1H), 7.54(d, J=8.8Hz, 1H), 7.42(d, J=8.0Hz, 2H), 6.98(d, J=8.0Hz, 1H), 6.55(m, 2H), 5.84(d, J=10.0Hz, 1H), 3.74(s, 3H), 3.06(m, 1H), 2.05(bs, 2H), 1.80(bs, 2H), 1.60(m, 4H), 1.37(s, 6H)

### 1.45 Synthesis of Formula 1-45 (1-(2-(4-cyclohexylphenyl)-1H-benzo[d]imidazol-5-yl)-3-(5-methoxy-2,2-dimethyl-2H-chromen-6-yl)urea)

The chromene-benzimidazole derivative of Formula 1-45 was synthesized as follows.

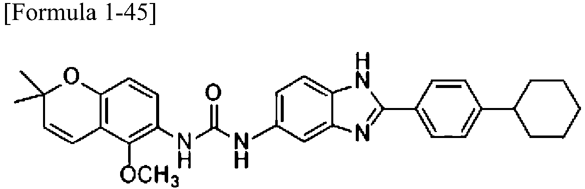

5-Methoxy-2,2-dimethyl-2H-chromene-6-carboxylic acid and 2-(4-cyclohexylphenyl)-1H-benzo[d]imidazol-5-amine were reacted in the same manner as in the method of Example 1-7 to obtain Compound 1-45.

Yield 79%
¹H NMR (400 MHz, DMSO-*d*₆) δ 12.70(bs, 1H), 9.23(bs, 1H), 8.07(m, 4H), 7.97(d, J=8.8Hz, 1H), 7.54(d, J=8.8Hz, 1H), 7.37(m, 3H), 6.98(d, J=10.4Hz, 1H), 6.55(m, 2H), 5.84(d, J=10.0Hz, 1H), 3.74(s, 3H), 1.83(m, 4H), 1.74(m, 1H), 1.48(m, 4H), 1.37(s, 6H), 1.23(m, 2H)

### 1.46 Synthesis of Formula 1-46 (1-(2-(4-((1r,3R,5S)-adamantan-1-yl)phenyl)-1H-benzo[d]imidazol-5-yl)-3-(5-methoxy-2,2-dimethyl-2H-chromen-6-yl)urea)

The chromene-benzimidazole derivative of Formula 1-46 was synthesized as follows.

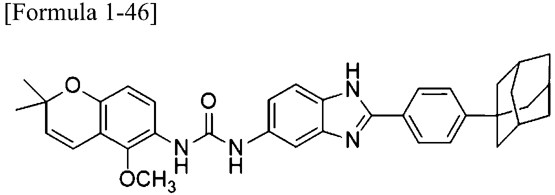

5-Methoxy-2,2-dimethyl-2H-chromene-6-carboxylic acid and 2-(4-((1r,3R,5S)-adamantan-1-yl)phenyl)-1H-benzo[d]imidazol-5-amine were reacted in the same manner as in the method of Example 1-7 to obtain Compound 1-46.

Yield 78%
¹H NMR (400 MHz, DMSO-*d*₆) δ 12.71(bs, 1H), 9.22(bs, 1H), 8.07(m, 3H), 7.97(s, 1H), 7.87(d, J=8.8Hz, 1H), 7.52(m, 3H), 6.98(d, J=8.8Hz, 1H), 6.55(m, 2H), 5.84(d, J=9.6Hz, 1H), 3.74(s, 3H), 2.08(s, 3H), 1.92(s, 6H), 1.76(s, 6H), 1.37(s, 6H)

### 1.47 Synthesis of Formula 1-47 (1-(2-(3-isopropylphenyl)-1H-benzo[d]imidazol-5-yl)-3-(5-methoxy-2,2-dimethyl-2H-chromen-6-yl)urea)

The chromene-benzimidazole derivative of Formula 1-47 was synthesized as follows.

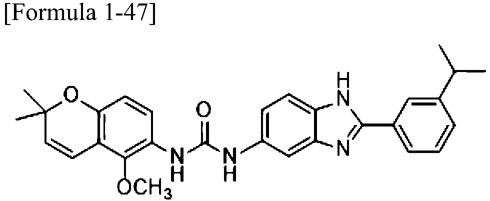

5-Methoxy-2,2-dimethyl-2H-chromene-6-carboxylic acid and 2-(3-isopropylphenyl)-1H-benzo[d]imidazol-5-amine were reacted in the same manner as in the method of Example 1-7 to obtain Compound 1-47.

Yield 79%
¹H NMR (400 MHz, DMSO-*d*₆) δ 12.76(bs, 1H), 9.23(bs, 1H), 8.11(s, 1H), 8.08-7.92(m, 3H), 7.84(m, 1H), 7.56(d, J=8.8Hz, 1H), 7.44(m, 1H), 7.35(m, 1H), 6.99(d, J=8.8Hz, 1H), 6.58(t, 2H), 5.84(d, J=9.6Hz, 1H), 3.74(s, 3H), 1.37(s, 6H), 1.29(d, J=6.8Hz, 6H)

### 1.48 Synthesis of Formula 1-48 (1-(5-methoxy-2,2-dimethyl-2H-chromen-6-yl)-3-(2-(4-nitrophenyl)-1H-benzo[d]imidazol-5-yl)urea)

The chromene-benzimidazole derivative of Formula 1-48 was synthesized as follows.

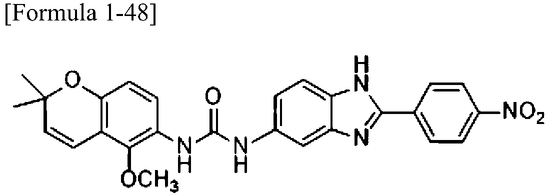

5-Methoxy-2,2-dimethyl-2H-chromene-6-carboxylic acid and 2-(4-nitrophenyl)-1H-benzo[d]imidazol-5-amine were reacted in the same manner as in the method of Example 1-7 to obtain Compound 1-48.

Yield 78%
¹H NMR (400 MHz, DMSO-*d*₆) δ 13.12(bs, 1H), 9.29(bs, 1H), 8.40(m, 4H), 8.13(s, 1H), 8.03(bs, 1H), 7.86(d, J=8.8Hz, 1H), 7.61(m, 1H), 7.06(m, 1H), 6.58(t, 2H), 5.84(d, J=10.0Hz, 1H), 3.74(s, 3H), 1.37(s, 6H)

### 1.49 Synthesis of Formula 1-49 (1-(2-(2,5-difluorophenyl)-1H-benzo[d]imidazol-5-yl)-3-(5-methoxy-2,2-dimethyl-2H-chromen-6-yl)urea)

The chromene-benzimidazole derivative of Formula 1-49 was synthesized as follows.

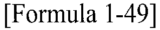

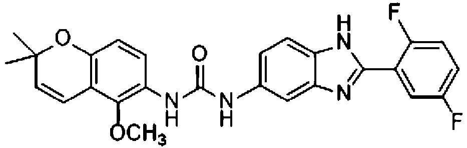

5-Methoxy-2,2-dimethyl-2H-chromene-6-carboxylic acid and 2-(2,5-difluorophenyl)-1H-benzo[d]imidazol-5-amine were reacted in the same manner as in the method of Example 1-7 to obtain Compound 1-49.

Yield 80%
¹H NMR (400 MHz, DMSO-*d*₆) δ 12.56(bs, 1H), 9.27(bs, 1H), 8.12(s, 1H), 8.04(s, 1H), 7.96(m, 1H), 7.87(d, J=8.8Hz, 1H), 7.61(d, J=8.8Hz, 1H), 7.50(m, 1H), 7.42(m, 1H), 7.08(d, J=8.8Hz, 1H), 6.55(t, 2H), 5.84(d, J=10.0Hz, 1H), 3.74(s, 3H), 1.37(s, 6H)

### 1.50 Synthesis of Formula 1-50 (1-(2-(2,3-difluorophenyl)-1H-benzo[d]imidazol-5-yl)-3-(5-methoxy-2,2-dimethyl-2H-chromen-6-yl)urea)

The chromene-benzimidazole derivative of Formula 1-50 was synthesized as follows.

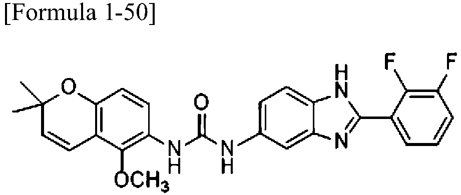

5-Methoxy-2,2-dimethyl-2H-chromene-6-carboxylic acid and 2-(2,3-difluorophenyl)-1H-benzo[d]imidazol-5-amine were reacted in the same manner as in the method of Example 1-7 to obtain Compound 1-50.

Yield 77%
¹H NMR (400 MHz, DMSO-*d*₆) δ 12.58(bs, 1H), 9.29(bs, 1H), 8.14(s, 1H), 8.04(s, 1H), 7.97(m, 1H), 7.87(d, J=9.2Hz, 1H), 7.62(m, 2H), 7.41(m, 1H), 7.07(d, J=8.8Hz, 1H), 6.55(t, 2H), 5.84(d, J=10.0Hz, 1H), 3.74(s, 3H), 1.37(s, 6H)

### 1.51 Synthesis of Formula 1-51 (1-(5-methoxy-2,2-dimethyl-2H-chromen-6-yl)-3-(2-(3-phenoxyphenyl)-1H-benzo[d]imidazol-5-yl)urea)

The chromene-benzimidazole derivative of Formula 1-51 was synthesized as follows.

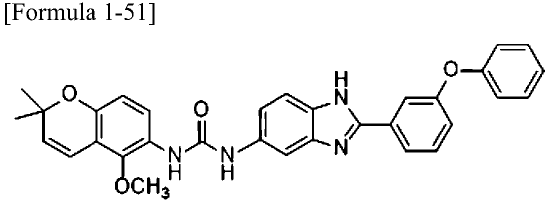

5-Methoxy-2,2-dimethyl-2H-chromene-6-carboxylic acid and 2-(3-phenoxyphenyl)-1H-benzo[d]imidazol-5-amine were reacted in the same manner as in the method of Example 1-7 to obtain Compound 1-51.

Yield 77%
¹H NMR (400 MHz, DMSO-*d*₆) δ 12.76(bs, 1H), 9.24(bs, 1H), 8.11(s, 1H), 7.98(s, 1H), 7.83(m, 2H), 7.68(s, 1H), 7.58(m, 2H), 7.43(m, 2H), 7.22(m, 1H), 7.12(m, 3H), 6.98(d, J=9.2Hz, 1H), 6.55(t, 2H), 5.84(d, J=10.0Hz, 1H), 3.74(s, 3H), 1.37(s, 6H)

### 1.52 Synthesis of Formula 1-52 (1-(2-([1,1'-biphenyl]-4-yl)-1H-benzold]imidazol-5-yl)-3-(5-methoxy-2,2-dimethyl-2H-chromen-6-yl)urea)

The chromene-benzimidazole derivative of Formula 1-52 was synthesized as follows.

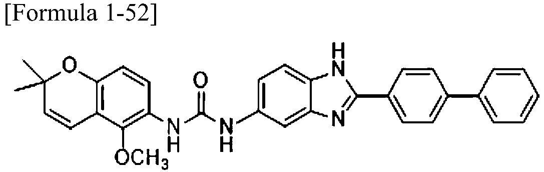

5-Methoxy-2,2-dimethyl-2H-chromene-6-carboxylic acid and 2-([1,1'-biphenyl]-4-yl)-1H-benzo[d]imidazol-5-amine were reacted in the same manner as in the method of Example 1-7 to obtain Compound 1-52.

Yield 73%
¹H NMR (400 MHz, DMSO-*d*₆) δ 12.86(bs, 1H), 9.25(bs, 1H), 8.26(m, 2H), 8.12(bs, 1H), 8.01(s, 1H), 7.88(m, 3H), 7.79(d, J=7.2Hz, 2H), 7.58(m, 3H), 7.49(m, 1H), 7.00(d, J=7.2Hz, 1H), 6.55(t, 2H), 5.84(d, J=10.0Hz, 1H), 3.74(s, 3H), 1.37(s, 6H)

### 1.53 Synthesis of Formula 1-53 (1-(2-(4-isobutylphenyl)-1H-benzo[d]imidazol-5-yl)-3-(5-methoxy-2,2-dimethyl-2H-chromen-6-yl)urea)

The chromene-benzimidazole derivative of Formula 1-53 was synthesized as follows.

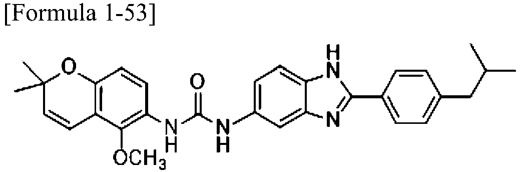

5-Methoxy-2,2-dimethyl-2H-chromene-6-carboxylic acid and 2-(4-isobutylphenyl)-1H-benzo[d]imidazol-5-amine were reacted in the same manner as in the method of Example 1-7 to obtain Compound 1-53.

Yield 79%
¹H NMR (400 MHz, DMSO-*d*₆) δ 12.70(bs, 1H), 9.22(bs, 1H), 9.10-8.02(m, 4H), 7.86(m, 1H), 7.54(d, J=8.8Hz, 1H), 7.33(d, J=8.0Hz, 2H), 6.97(d, J=8.8Hz, 1H), 6.55(t, 2H), 5.84(d, J=10.0Hz, 1H), 3.74(s, 3H), 2.52(d, J=8.0, Hz, 2H), 1.91(m, 1H), 1.37(s, 6H), 0.91(d, J=6.4Hz, 6H)

### Example 2. Measurement of Anticancer Activity and Confirmation of Mechanism of Action of Chromene-Benzimidazole Derivatives

### 2.1 Screening of Cell Viability of Chromene-Benzimidazole Derivatives

MDA-MB-231 (Cell seeding numbers: 0.8(M231) x 10⁴ cells/wells (confluency ≧ 25%), which is a human triple-negative breast cancer (TNBC) cell line, and JIMT-1 (Cell seeding numbers: 0.8(JIMT) x 10⁴ cells/wells (confluency ≧ 25%)), which is a HER2 positive breast cancer (HER2+ BC) cell line, were used in the experiment.

The above cell lines were cultured in Dulbecco's modified Eagle's medium (DMEM) containing 10% fetal bovine serum (FBS), streptomycin-penicillin (100 U/mL), and Fungizone (0.625 *µ*g/mL) at 5% CO₂ and 37°C.

The human breast cancer cell lines MDA-MB-231 and JIMT-1 were treated with chromene-benzimidazole derivatives of Formulas 1-1 to 1-53 at various concentrations of 0, 1, and 5 µM, respectively, for 72 hours, and then cell viability was measured using the MTS assay technique. In the MTS assay, cells were attached to a 96-well plate for 24 hours, treated with the chromene-benzimidazole derivative for 72 hours, developed with MTS (3-(4,5-dimethylthiazol-2-yl)-5-(3-carboxymethoxyphenyl)-2-(4-sulfophenyl)-2H-tetrazolium) for 4 hours, and then the absorbance was measured at 490 nm using a Spectramax Plus384 microplate analyzer.

The measured results are shown in FIGS. 1 to 6. As a result, it was confirmed that most of the chromene-benzimidazole derivatives of Formulas 1-1 to 1-53 inhibited cell viability in a concentration-dependent manner in the human breast cancer cell lines MDA-MB-231 and JIMT-1.

### 2.2 Confirmation of Apoptosis Effect of Chromene-Benzimidazole Derivatives

After treating the triple-negative breast cancer cell lines MDA-MB-231 and BT549 with the chromene-benzimidazole derivative Compound (1-1) by concentration (0, 0.01, 0.05, 0.1, 0.5, 1, 5, 10, 20, and 40 µM) for 72 hours, concentration-dependent cell viability (MTS assay) inhibition and IC50 values according to the cell viability inhibition were confirmed and shown in FIGS. 7A and 7B. For the compound of Formula 1-1, IC50 values of 5.659 µM and 8.491 µM were observed in MDA-MB-231 and BT549, respectively.

After treating the MDA-MB-231 and BT549 cell lines with the 1-1 compound by concentration (0, 5, 10, and 20 µM) for 72 hours, morphological changes of cells were observed using an optical microscope, which is shown in FIG. 7C. It was confirmed that apoptosis was induced in a concentration-dependent manner.

The degree of apoptosis of MDA-MB-231 and BT549 cells by the compound of Formula 1-1 was confirmed by a flow cytometer through Annexin V/PI staining, which is shown in FIG. 7D. It was confirmed that early and late apoptosis were induced in both cancer cells.

### 2.3 Confirmation of Mechanism of Action of Chromene-Benzimidazole Derivatives

As described above, the chromene-benzimidazole derivative of the disclosure is designed to strongly bind to a C-terminal domain of Hsp90 and simultaneously bind between tubulin polymers to have an ability to inhibit a polymerization reaction. To verify such a dual target effect, an experiment was performed as follows.

In order to evaluate the ability to inhibit the C-terminal domain of Hsp90, it was attempted to evaluate the ability to inhibit Hsp90 C-terminal activity through analysis of binding force between the 1-1 compound, a C-terminal Hsp90 inhibitor Novobiocin, and an N-terminal inhibitor Onalespib, and a C recombinant protein. The experimental results are shown in FIG. 8A. It was confirmed that the compound of Formula 1-1 of the disclosure lowered C-terminal HSP90 activity to less than half compared to the existing Novobiocin, and thus the inhibitory ability was significantly excellent.

In addition, after treating the triple-negative breast cancer cell line MDA-MB-231 with the compound of Formula 1-1 by concentration (0, 5, 10, and 20 µM) for 72 hours, decreases in protein expression and phosphorylation of STAT3, c-MET, ERK, and AKT known as client proteins of HSP90 were confirmed using a Western blot technique, which is shown in FIG. 8B.

Subsequently, after treating the MDA-MB-231 cell line with the compound of Formula 1-1 by concentration (0, 5, 10, and 20 µM) for 72 hours, degradation of tubulin and expression of apoptosis-related proteins were confirmed by Western blot, which is shown in FIG. 8C. It was confirmed that the compound of the disclosure could inhibit a tubulin polymerization reaction.

Finally, the BT549 cell line was treated with the compound of Formula 1-1 by concentration (0, 5, 10, and 20 µM) for 72 hours, stained with propidium iodide, and then the DNA content of the cancer cells was analyzed using flow cytometry to confirm G2/M phase cell cycle arrest of the chromene-benzimidazole derivative, which is shown in FIG. 8D.

### Example 3. Screening of Effective Substances of Chromene-Benzimidazole Derivatives and Confirmation of Anticancer Activity by Cancer Type

### 3.1 Derivation of Effective Substance of Chromene-Benzimidazole Derivatives

The triple-negative breast cancer cell lines MDA-MB-231 and JIMT-1 were treated with the chromene-benzimidazole derivative compounds (1-13, 1-19, and 1-22) by concentration (0, 0.01, 0.05, 0.1, 0.25, 0.5, 1, 2, and 5 µM) for 72 hours. Detailed treatment conditions were the same as disclosed in Example 2.1. Concentration-dependent cell viability (MTS assay) inhibition and IC50 values according to the cell viability inhibition were confirmed and shown in FIG. 9. It was confirmed that IC50 values for Compounds 1-13, 1-19, and 1-22 of the disclosure were 0.154, 1.06, and 0.276 µM, respectively, which were very excellent.

Subsequently, an experiment was performed as follows to confirm the targeting ability for Compounds 1-13 and 1-22. After treating the MDA-MB-231 cell line with the 1-22 and 1-13 compounds at a concentration of 0.5 µM for 72 hours, morphological changes of cells were observed using an optical microscope, and micrographs are shown in FIG. 10A. Typical morphological changes of cell cycle arrest and apoptosis could be confirmed. In addition, after treating MDA-MB-231 with the 1-22 and 1-13 compounds at a concentration of 0.5 µM for 72 hours, expression of HSP90 chaperon complexes (HSP90, HSP70, HSP27, HSF-1), HSP90 client proteins (EGFR, AKT, JAK2, STAT3), Tubulin targeting ability (Tubulin, mitosis marker p-His H3), and apoptosis-related factors (Cleaved caspase-7, Survivin, Mcl-1) proteins was confirmed using a Western blot technique, which is shown in FIG. 10B.

### 3.2 Confirmation of Anticancer Activity of Chromene-Benzimidazole Derivatives by Cancer Type

After treating a hematologic cancer cell line HL-60, a liver cancer cell line HepG2, a lung cancer cell line H1299, a colorectal cancer cell line HCT116, and an ovarian cancer cell line SKOV3 with the chromene-benzimidazole derivative compound 1-22 by concentration (0, 0.1, 0.5, 1, 5, and 10 µM) for 72 hours, concentration-dependent cell viability (MTS assay) inhibition and IC50 values according to the cell viability inhibition were confirmed and shown in FIG. 11. The same experiment was performed for Compound 1-13, which is shown in FIG. 12. In other cancer type cell lines excluding breast cancer, effective cell viability inhibition could be confirmed for both compounds.

### 3.3 Derivation of Additional Effective Substances of Chromene-Benzimidazole Derivatives

After treating the triple-negative breast cancer cell line MDA-MB-231 with the chromene-benzimidazole derivative compounds (1-9, 1-10, 1-11, 1-15, 1-16, and 1-17) by concentration (0, 0.01, 0.05, 0.1, 0.25, 0.5, 1, 2, and 5 µM) for 72 hours, concentration-dependent cell viability (MTS assay) inhibition and IC50 values according to the cell viability inhibition were confirmed and shown in FIG. 13. Very excellent IC50 values of 0.273, 0.829, 0.335, 0.452, 0.96, and 0.586 µM could be confirmed for Compounds 1-9, 1-10, 1-11, 1-15, 1-16, and 1-17, respectively.

### Comparative Example 1. Comparative Experiment with Analogous Structure Compound

A comparative experiment was performed with the following Reference compound in the prior literature having structural similarity to the compound of the disclosure.

### [Comparative Experimental Compound]

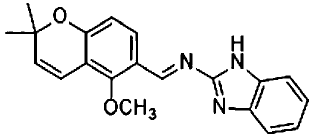

The Ref. compound is a compound in which chromene and benzimidazole are combined similarly to the disclosure, but there is a difference in that the Ref. compound has the 2-carbon of benzimidazole connected to chromene through an imine, whereas the disclosure has the 5-carbon of benzimidazole connected to chromene through a urea. After treating the MDA-MB-231 and JIMT-1 cell lines with the chromene-benzimidazole derivative compounds (1-19 and 1-22) of the disclosure or the Ref. compound by concentration (0, 2, 5, and 10 µM) for 72 hours, effects on cell viability were compared through an MTS assay technique and shown in FIG. 14. Unlike the chromene-benzimidazole derivative of the disclosure, the Ref. compound known in the prior literature did not inhibit cell viability at all in both cancer cells.

While the embodiments have been described with reference to the limited drawings, it will be apparent to one of ordinary skill in the art that various alterations and modifications can be made from the above description. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A chromene-benzimidazole derivative represented by Formula 1 or a pharmaceutically acceptable salt thereof: 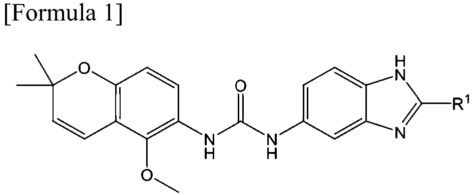 wherein in Formula 1,
R¹ is any one selected from the group consisting of hydrogen, halogen, a substituted or unsubstituted linear or branched C₁-C₆ alkyl group, a substituted or unsubstituted C₃-C₂₀ cycloalkyl group, a substituted or unsubstituted C₃-C₂₀ heterocycloalkyl group, a substituted or unsubstituted C₃-C₂₀ aryl group, and a substituted or unsubstituted C₃-C₂₀ heteroaryl group,
when the linear or branched alkyl group is substituted, the alkyl group is substituted with halogen, and
when the cycloalkyl group, heterocycloalkyl group, aryl group, or heteroaryl group is substituted, the group is substituted with at least one selected from the group consisting of linear or branched C₁-C₆ alkyl, C₁-C₁₀ cycloalkyl, halogen, hydroxy, cyano, trifluoromethyl, trifluoromethoxy, alkoxy, amino, nitro, phenyl, phenoxy, benzoyloxymethyl, indole, methylpiperazine, and benzoyl.

2. The chromene-benzimidazole derivative or pharmaceutically acceptable salt thereof of claim 1,
wherein in Formula 1,
the substituted or unsubstituted linear or branched C₁-C₆ alkyl group of R¹ is a substituted or unsubstituted methyl group, and when the methyl group is substituted, the methyl group is substituted with halogen,
the substituted or unsubstituted C₃-C₂₀ cycloalkyl group of R¹ is an unsubstituted cyclohexyl group,
the substituted or unsubstituted C₃-C₂₀ heterocycloalkyl group of R¹ is a methylpiperazine group,
the substituted or unsubstituted C₃-C₂₀ aryl group of R¹ is a substituted or unsubstituted phenyl group, and when the phenyl group is substituted, the phenyl group is substituted with at least one selected from the group consisting of linear or branched C₁-C₆ alkyl, C₁-C₁₀ cycloalkyl, halogen, hydroxy, cyano, trifluoromethyl, trifluoromethoxy, alkoxy, amino, nitro, phenyl, phenoxy, benzoyloxymethyl, indole, methylpiperazine, and benzoyl, and
the substituted or unsubstituted C₃-C₂₀ heteroaryl group of R¹ is any one selected from the group consisting of a pyridyl group, a thiophenyl group, and a thiazolyl group.

3. The chromene-benzimidazole derivative or pharmaceutically acceptable salt thereof of claim 1,
wherein in Formula 1,
R¹ is a substituted or unsubstituted phenyl group, and
when the phenyl group is substituted, the phenyl group is substituted with at least one selected from the group consisting of methyl, propyl, isopropyl, isobutyl, tert-butyl, cyclopropyl, cyclopentyl, cyclohexyl, adamantyl, F, Cl, hydroxy, cyano, trifluoromethyl, trifluoromethoxy, methoxy, pentoxy, N,N-dimethylamino, N,N-diethylamino, nitro, phenyl, phenoxy, benzoyloxymethyl, indole, methylpiperazine, and benzoyl.

4. The chromene-benzimidazole derivative or pharmaceutically acceptable salt thereof of claim 1, wherein the chromene-benzimidazole derivative represented by Formula 1 is any one selected from the group consisting of the following compounds: 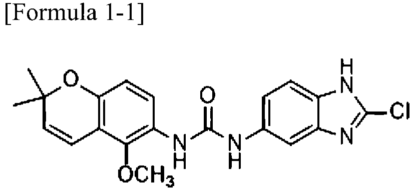 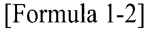 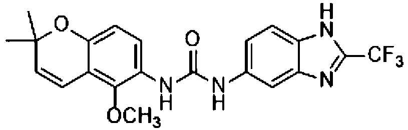 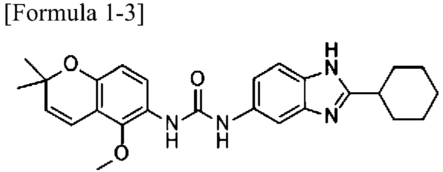 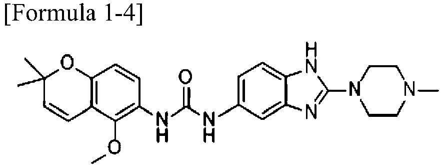 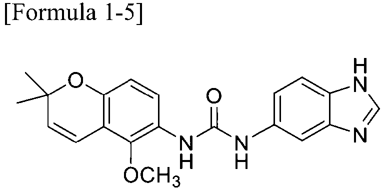 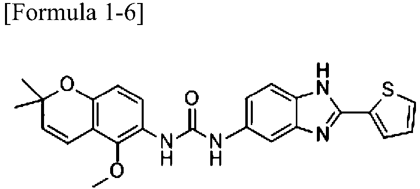 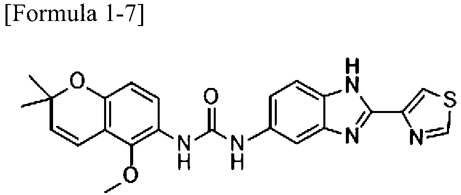 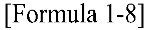 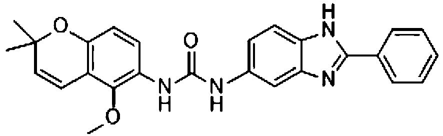 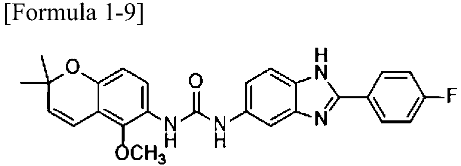 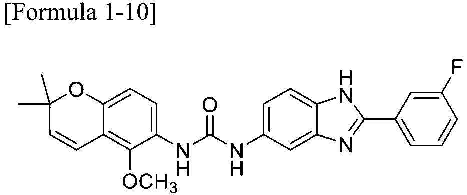 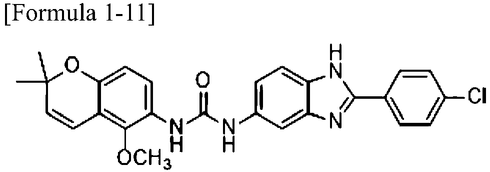 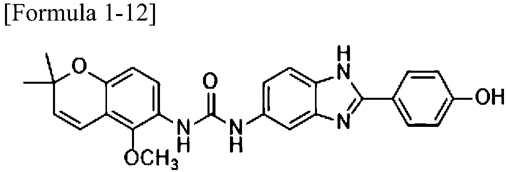 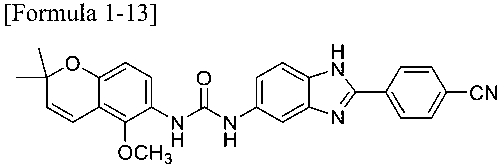 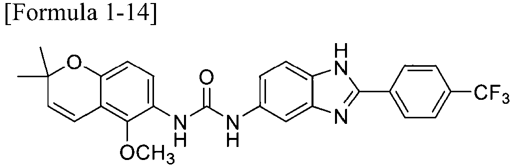 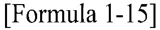 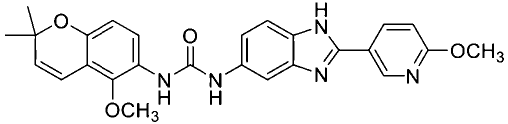 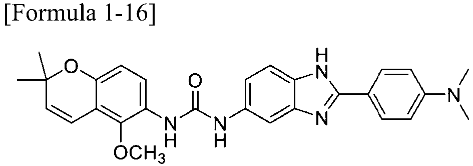 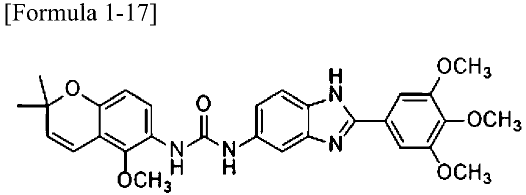 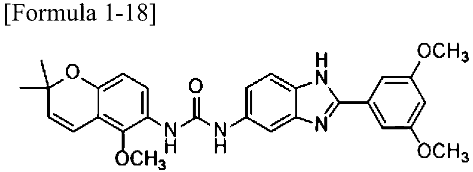 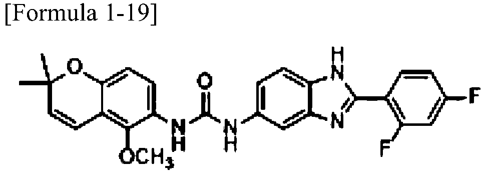 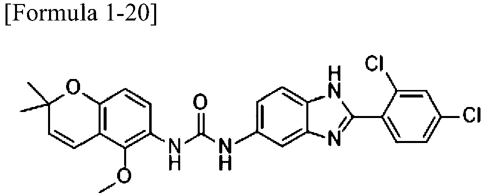 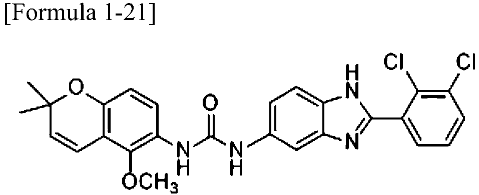 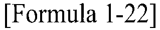 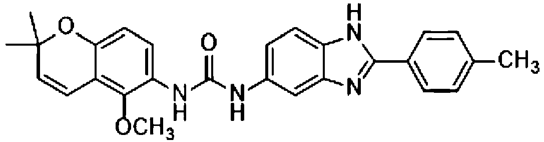 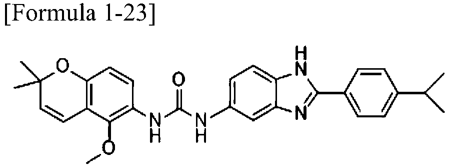 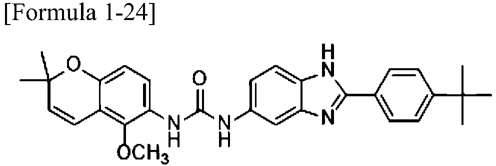 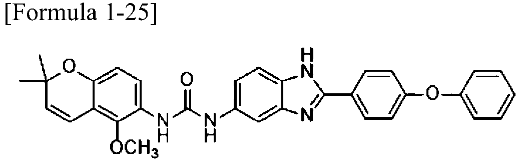 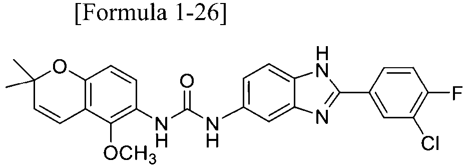 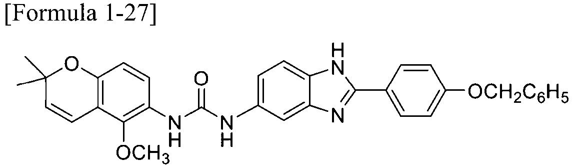 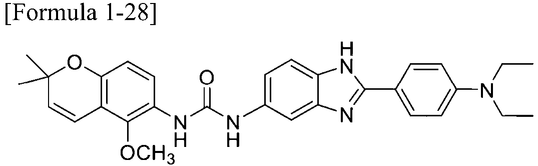 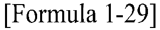 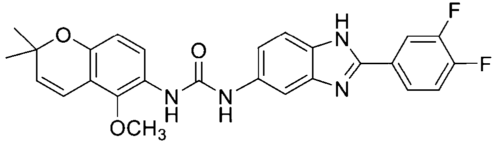 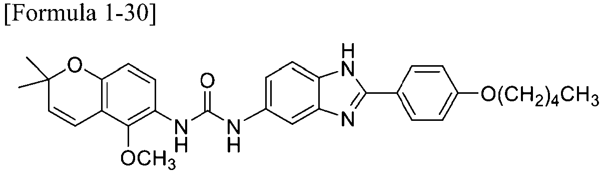 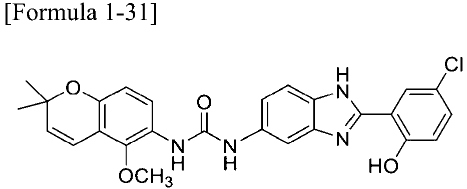 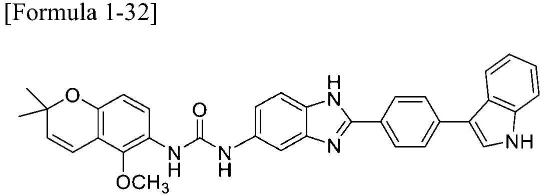 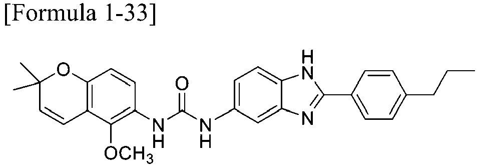 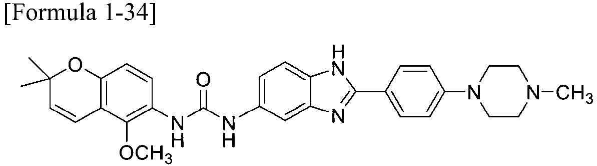 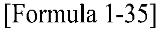 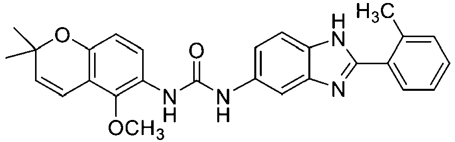 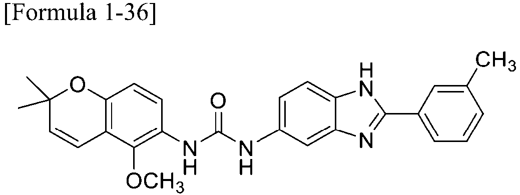 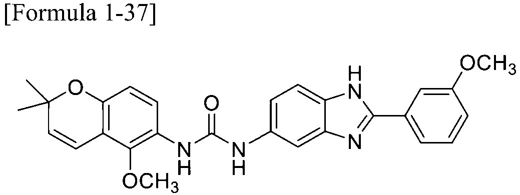 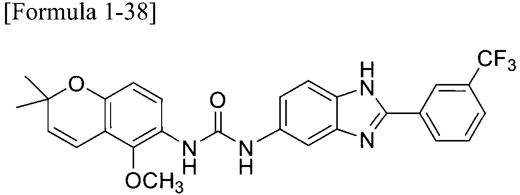 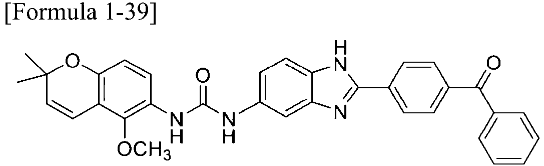 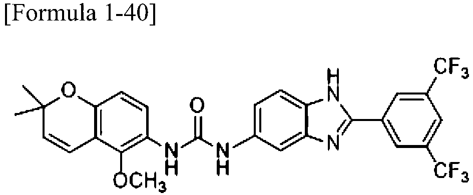 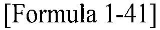 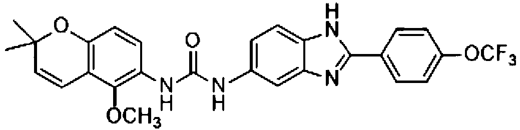 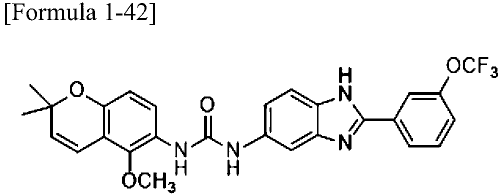 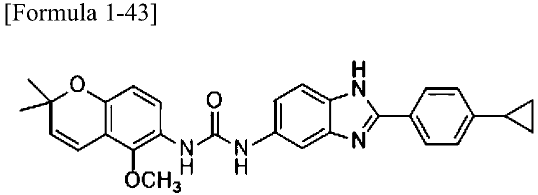 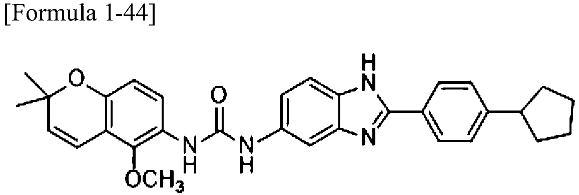 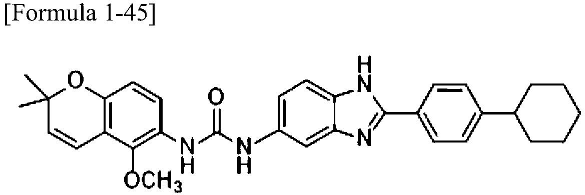 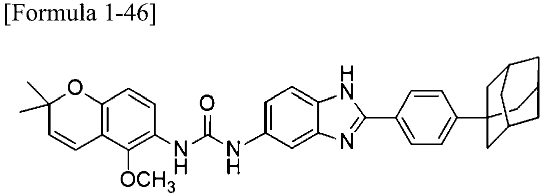 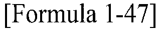 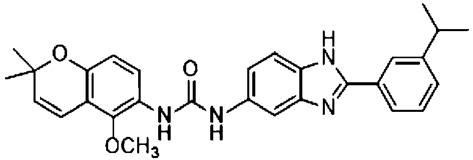 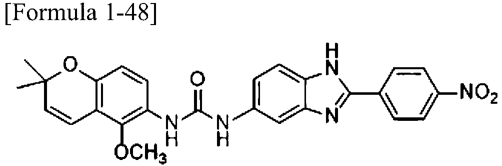 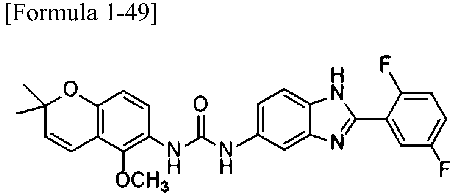 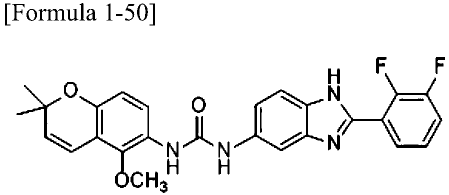 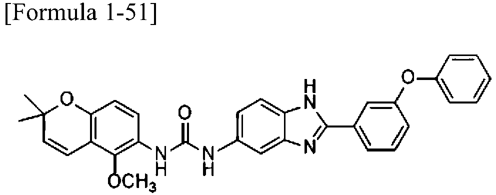 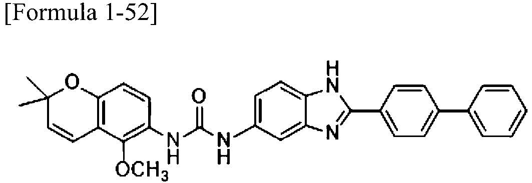 and

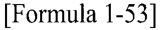

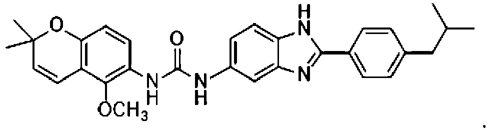

5. The chromene-benzimidazole derivative or pharmaceutically acceptable salt thereof of claim 1, wherein the chromene-benzimidazole derivative represented by Formula 1 is any one selected from the group consisting of the following compounds: 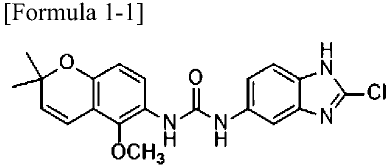 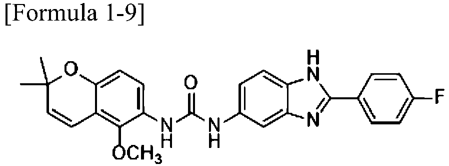 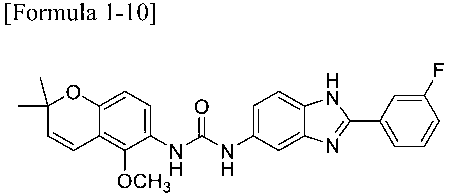 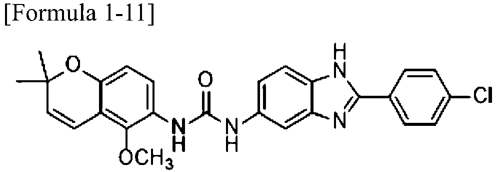 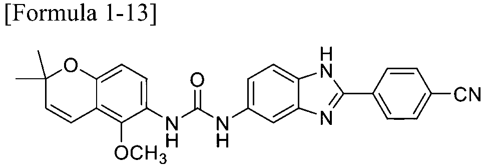 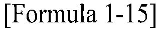 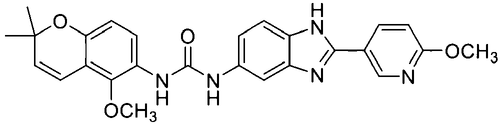 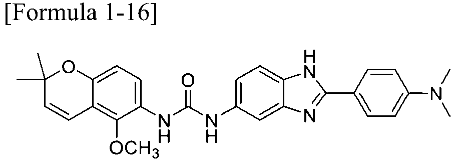 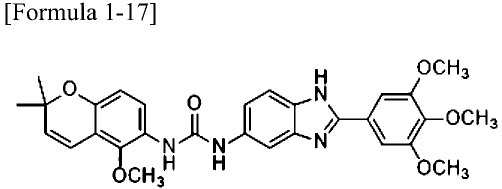 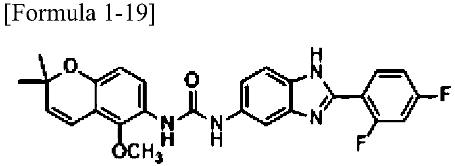 and

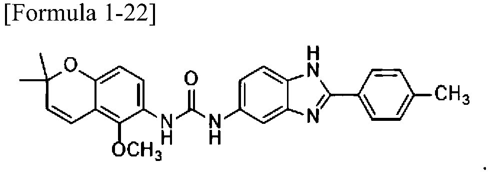

6. The chromene-benzimidazole derivative or pharmaceutically acceptable salt thereof of claim 1, wherein the chromene-benzimidazole derivative has both an effect of inhibiting tubulin polymerization and an effect of inhibiting a C-terminal domain of Hsp90.

7. The chromene-benzimidazole derivative or pharmaceutically acceptable salt thereof of claim 1, wherein the pharmaceutically acceptable salt of the chromene-benzimidazole derivative is at least one selected from the group consisting of a hydrochloride salt, a bromate salt, a sulfate salt, a phosphate salt, a nitrate salt, a citrate salt, an acetate salt, a lactate salt, a tartrate salt, a maleate salt, a gluconate salt, a succinate salt, a formate salt, a trifluoroacetate salt, an oxalate salt, a fumarate salt, a glutarate salt, an adipate salt, a methanesulfonate salt, a benzenesulfonate salt, a paratoluenesulfonate salt, a camphorsulfonate salt, a sodium salt, a potassium salt, a lithium salt, a calcium salt, and a magnesium salt.

8. A pharmaceutical composition for preventing or treating cancer, comprising the chromene-benzimidazole derivative of any one of claims 1 to 7, or a pharmaceutically acceptable salt thereof as an active ingredient.

9. The pharmaceutical composition for preventing or treating cancer of claim 8, wherein the pharmaceutical composition induces apoptosis by cell cycle arrest of cancer cells.

10. The pharmaceutical composition for preventing or treating cancer of claim 8, wherein the pharmaceutical composition induces denaturation and degradation of an oncoprotein.

11. The pharmaceutical composition for preventing or treating cancer of claim 8, wherein the cancer is at least one selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, hematologic cancer, thymic cancer, urethral cancer, and bronchial cancer.

12. The pharmaceutical composition for preventing or treating cancer of claim 8, wherein the cancer is triple-negative breast cancer.
